(19) **Europäisches Patentamt European Patent Office Office européen des brevets**

(11) **EP 4 179 318 B1**

(12) **FASCICULE DE BREVET EUROPEEN**

(45) Date de publication et mention de la délivrance du brevet:
**10.04.2024 Bulletin 2024/15**

(21) Numéro de dépôt: **21746535.0**

(22) Date de dépôt: **08.07.2021**

(51) Classification Internationale des Brevets (IPC):
**G01N 33/49** *(2006.01)*    **A61M 1/02** *(2006.01)*
**G01N 21/25** *(2006.01)*    **G01N 21/85** *(2006.01)*

(52) Classification Coopérative des Brevets (CPC):
**G01N 33/4915; A61M 1/02; A61M 1/3609; G01N 21/255; G01N 21/85;** A61M 2205/3306; A61M 2230/207; G01N 21/3151; G01N 2201/069

(86) Numéro de dépôt international:
**PCT/FR2021/051272**

(87) Numéro de publication internationale:
**WO 2022/008850 (13.01.2022 Gazette 2022/02)**

(54) **APPAREIL ET PROCÉDÉ DE DÉTERMINATION DU TAUX D'HÉMOGLOBINE OU D'HÉMATOCRITE D'UN LIQUIDE EN CIRCULATION**

VORRICHTUNG UND VERFAHREN ZUR BESTIMMUNG DES HÄMOGLOBIN- ODER HÄMATOKRITWERTES EINER STRÖMENDEN FLÜSSIGKEIT

DEVICE AND METHOD FOR DETERMINING THE HAEMOGLOBIN OR HAEMATOCRIT LEVEL OF A FLOWING LIQUID

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats de validation désignés:
**MA**

(30) Priorité: **08.07.2020 FR 2007201**
**08.07.2020 FR 2007200**

(43) Date de publication de la demande:
**17.05.2023 Bulletin 2023/20**

(73) Titulaire: **I-Sep**
**44200 Nantes (FR)**

(72) Inventeurs:
• **CHOLLET, Stéphane**
**44240 LA CHAPELLE SUR ERDRE (FR)**
• **COULON, Gaëtan**
**56870 BADEN (FR)**
• **DECOUTURE, Benoît**
**44000 NANTES (FR)**
• **PICOT, Sylvain**
**69300 CALUIRE ET CUIRE (FR)**

• **FOREST-VILLEGAS, Patricia**
**69740 GENAS (FR)**
• **GADRAT, Francis**
**33200 BORDEAUX (FR)**

(74) Mandataire: **Regimbeau**
**20, rue de Chazelles**
**75847 Paris Cedex 17 (FR)**

(56) Documents cités:
**WO-A1-2008/136548    WO-A1-2012/068416**
**US-A1- 2007 060 810**

• **EKUNI SHOTA ET AL: "Noninvasive and Continuous Hematocrit Measurement by Optical Method without Calibration", ELECTRONICS AND COMMUNICATIONS IN JAPAN. , vol. 99, no. 9 12 août 2016 (2016-08-12), pages 37-46, XP055787409, US ISSN: 1942-9533, DOI: 10.1002/ecj.11847 Extrait de l'Internet: URL:https://api.wiley.com/onlinelibrary/td m/v1/articles/10.1002%2Fecj.11847 cité dans la demande**

EP 4 179 318 B1

**Description**

DOMAINE TECHNIQUE

La présente divulgation concerne un appareil et une méthode pour la détermination d'un paramètre sanguin d'un liquide en circulation, en particulier pour la détermination du taux d'hémoglobine et/ou du taux d'hématocrite du liquide en circulation. La présente divulgation trouve une application particulièrement avantageuse pour des applications médicales, par exemple pour l'analyse de liquide hémorragique en circulation dans un tube.

ETAT DE LA TECHNIQUE

Il existe de nombreuses applications médicales pour lesquelles le suivi des paramètres sanguins permettant de qualifier le liquide hémorragique du patient est nécessaire. Suivre l'évolution du taux d'hémoglobine et/ou du taux d'hématocrite d'un liquide hémorragique peut par exemple être utile dans certaines interventions, notamment chirurgicales. En particulier, lorsque le liquide hémorragique d'un patient doit subir un traitement spécifique, il est avantageux de pouvoir suivre en continu l'évolution de ces paramètres sanguins.

Un exemple particulier et non limitatif où il est utile de pouvoir suivre l'évolution des paramètres sanguins est le traitement de liquide hémorragique en vue d'une autotransfusion de sang chez un patient. L'autotransfusion ou transfusion autologue, à savoir la transfusion chez un patient de son propre sang, est de plus en plus pratiquée lors d'interventions chirurgicales, puisqu'elle évite les incompatibilités qui peuvent survenir lors de transfusions homologues ou allogéniques. L'autotransfusion évite de plus la transmission de maladies infectieuses.

Pour un bon fonctionnement de ces systèmes de traitement de liquide hémorragique, il est impératif de pouvoir suivre en temps réel l'évolution de la concentration en hémoglobine ou du taux d'hématocrite dans le liquide en cours de traitement puisque l'évolution des paramètres sanguins de ce liquide peut permettre de piloter le système de traitement. Une des difficultés réside dans le fait que le liquide dont on souhaite connaitre le taux d'hémoglobine et/ou d'hématocrite est en circulation, en général dans des tubulures souples, ce qui vient complexifier la détection. En outre, il est nécessaire d'appliquer des méthodes de détection spécifiques pour compenser les pertes de sensibilité de la détection dues au mouvement du liquide. Par ailleurs, les systèmes de traitement de liquide hémorragique (notamment en vue d'autotransfusion) étant en général utilisé dans des situations d'urgence, il est important que l'ensemble du système puisse être utilisable immédiatement, en évitant dans la mesure du possible toute étape de calibration préalable, y compris s'agissant du composant permettant de déterminer le taux d'hémoglobine et/ou d'hématocrite du liquide hémorragique à traiter.

Dans l'article intitulé « Noninvasive and Continuous Hematocrit Measurement by Optical Method without Calibration » publié par SHOTA EKUNI et YOSHIYUKI SANKAI dans « Electronics and Communications in Japan, Vol. 99, No. 9, 2016 », il a été proposé une méthode et un système de détection optique pour la détermination du taux d'hématocrite d'un liquide en circulation dans un tube et évitant une calibration préalable du système de détection. Le système optique proposé est constitué de deux ensembles émetteur-récepteur fonctionnant en rétrodiffusion, chaque ensemble émetteur-récepteur étant agencé sur un support respectif. Les deux supports sont prévus pour être attachés l'un à l'autre tout en venant entourer une portion tubulaire à travers laquelle circule le liquide dont le taux d'hématocrite est à déterminer, sans toutefois venir déformer cette portion tubulaire. Les deux ensembles émetteur-récepteur fonctionnent selon des longueurs d'onde différentes correspondant à des points isobestiques de l'hémoglobine, à savoir à 810 nm et 1300 nm, et de manière alternée pour éviter des interférences dans les mesures. Selon cet article, la distance entre l'émetteur et le récepteur influe de manière importante sur la fiabilité de la détermination du taux d'hématocrite et il apparait donc nécessaire de la garder la plus faible possible (inférieure à 4 mm). Cela entraine en pratique des contraintes importantes en termes de fabrication et d'application.

EXPOSE DE L'INVENTION

Un but de la présente invention est de proposer un appareil de détermination d'un paramètre sanguin tel que la concentration en hémoglobine (appelée également taux d'hémoglobine) et/ou le taux d'hématocrite pouvant être utilisé pour un liquide en circulation dans une portion tubulaire ayant un diamètre quelconque, notamment un tube souple utilisé en milieu médical.

Un autre but de l'invention est de proposer un appareil de détermination du taux d'hémoglobine et/ou du taux d'hématocrite d'un liquide en circulation dans une portion tubulaire présentant une fiabilité accrue, et permettant notamment des mesures dans une gamme large des taux. Par exemple, un but est de permettre des mesures de taux d'hématocrite aussi bien pour des taux d'hématocrite faibles, c'est-à-dire inférieurs ou égaux à 30%, que pour des taux d'hématocrite élevés, c'est-à-dire supérieurs à 30%. En particulier, un but de la présente invention est de proposer un appareil permettant de déterminer des taux d'hématocrite au moins compris dans la gamme de 5% à 60%, et notamment entre 20% et 50%.

Un autre but de la présente invention est de proposer un appareil de détermination du taux d'hémoglobine et/ou du taux d'hématocrite d'un liquide en circulation dans une portion tubulaire qui peut être positionné sur cette portion tubulaire de manière simple, sans avoir notamment à modifier cette portion tubulaire, et sans avoir à stopper la circulation du liquide le cas échéant. Avantageusement, l'appareil de détermination du taux d'hémoglobine et/ou du taux d'hématocrite proposé peut être directement utilisé sur un système de traitement d'un liquide, par exemple un système de traitement de liquide hémorragique pour de l'autotransfusion, en utilisant les tubulures préexistantes dans le système de traitement, sans avoir à démonter les éléments de ce système de traitement notamment.

Encore un but de la présente invention est de proposer un appareil de détermination du taux d'hémoglobine et/ou du taux d'hématocrite pouvant être utilisé pour un liquide circulant dans une portion tubulaire à un débit important (typiquement supérieur à 1000 ml/min, par exemple de l'ordre de 2000 ml/min) sans toutefois venir substantiellement perturber le flux de ce liquide en circulation, pour éviter de possibles effets néfastes sur le liquide, par exemple pour éviter de créer une hémolyse pour une circulation de liquide hémorragique.

Un autre but de la présente invention est de proposer un procédé de détermination du taux d'hémoglobine et/ou du taux d'hématocrite d'un liquide en circulation dans une portion tubulaire, fiable, simple à mettre en oeuvre, et permettant des mesures dans une gamme large de taux, en particulier aussi bien pour des taux d'hématocrite faibles, c'est-à-dire inférieurs ou égaux à 30%, que pour des taux d'hématocrite élevés, c'est-à-dire supérieurs à 30%. En particulier, un but de la présente invention est de proposer un procédé permettant de déterminer des taux d'hématocrite au moins compris dans la gamme de 5% à 60%, et notamment entre 20% et 50%.

A cette fin, on propose un appareil de détermination du taux d'hématocrite et/ou du taux d'hémoglobine d'un liquide en circulation dans une portion tubulaire, comprenant :

deux ensembles émetteur-récepteur, chaque ensemble émetteur-récepteur comprenant une source lumineuse et un capteur lumineux prévus pour être agencés de part et d'autre de la portion tubulaire au niveau d'une zone de circulation du liquide pour une mesure en transmission, de préférence à travers des parois incurvées de la portion tubulaire ;
la source lumineuse de chacun des deux ensembles émetteur-récepteur étant configurée pour émettre des faisceaux lumineux selon une longueur d'onde d'émission choisie pour correspondre à un point isobestique de l'hémoglobine ;
chaque ensemble émetteur-récepteur comprenant en outre un système de collimation du faisceau lumineux émis depuis la source lumineuse correspondante en direction du capteur lumineux correspondant.

On propose également un appareil de détermination du taux d'hématocrite et/ou du taux d'hémoglobine d'un liquide en circulation dans une portion tubulaire, comprenant :

deux ensembles émetteur-récepteur, chaque ensemble émetteur-récepteur comprenant une source lumineuse et un capteur lumineux prévus pour être agencés de part et d'autre de la portion tubulaire au niveau d'une zone de circulation du liquide pour une mesure en transmission, de préférence à travers des parois incurvées de la portion tubulaire ;
la source lumineuse de chacun des deux ensembles émetteur-récepteur étant configurée pour émettre des faisceaux lumineux selon une longueur d'onde d'émission choisie pour correspondre à un point isobestique de l'hémoglobine ;
un système de traitement programmé pour déterminer le taux d'hématocrite et/ou du taux d'hémoglobine du liquide en fonction des signaux lumineux reçus par les capteurs lumineux des ensembles émetteur-récepteur ; et
un système de contrôle comprenant des moyens de modification de la puissance émise par les sources lumineuses, le système de contrôle étant programmé pour modifier la puissance d'émission des sources lumineuses en fonction du taux d'hématocrite et/ou du taux d'hémoglobine déterminé pour le liquide.

Des aspects préférés mais non limitatifs de l'un ou l'autre de ces appareils, pris seuls ou en combinaison, sont les suivants :

- l'appareil comprend un ensemble support sur lequel sont montés les deux ensembles émetteur-récepteur, l'ensemble support étant prévu pour être positionné autour de la portion tubulaire ;
- les sources lumineuses respectives des deux ensembles émetteur-récepteur sont configurées pour émettre des faisceaux lumineux à deux longueurs d'onde d'émission différentes ;
- au moins une des sources lumineuses des ensembles émetteur-récepteur est configurée pour émettre des faisceaux lumineux selon une longueur d'onde d'émission choisie pour une absorption des faisceaux lumineux sensiblement identique dans de l'eau ou dans du plasma ;
- au moins un, et de préférence chaque, système de collimation comprend un ensemble de lentille(s) amont ayant un plan focal et étant positionné entre la source lumineuse et le capteur lumineux correspondants du côté de la source lumineuse par rapport à la portion tubulaire, la source lumineuse étant positionnée à plus ou moins 10 mm du plan focal de l'ensemble de lentille(s) amont, et de préférence dans plan focal de l'ensemble de lentille(s) amont ;
- au moins un, et de préférence chaque, système de collimation comprend un ensemble de lentille(s) aval ayant un plan focal et étant positionné entre la source lumineuse et le capteur lumineux correspondants du côté du capteur lumineux par rapport à la portion tubulaire, le capteur lumineux étant positionné à plus ou moins 10 mm du plan focal de l'ensemble de lentille(s) aval, et de préférence dans plan focal de l'ensemble de lentille(s) aval ;

- au moins un, et de préférence chaque, système de collimation comprend un ensemble de lentille(s) aval ayant un plan focal et étant positionné entre la source lumineuse et le capteur lumineux correspondants du côté du capteur lumineux par rapport à la portion tubulaire, l'ensemble de lentille(s) aval est positionné de sorte que les faisceaux lumineux en sortie de la paroi de sortie de la portion tubulaire convergent à plus ou moins 10 mm du plan focal de l'ensemble de lentille(s) aval, et de préférence dans plan focal de l'ensemble de lentille(s) aval ;

- au moins un, et de préférence chaque, système de collimation comprend un diaphragme amont positionné entre la source lumineuse et le capteur lumineux correspondants du côté de la source lumineuse par rapport à la portion tubulaire, le diaphragme amont étant prévu pour laisser passer une portion centrale des faisceaux lumineux émis par la source lumineuse en direction du capteur lumineux et pour stopper une portion périphérique des faisceaux lumineux émis par la source lumineuse ;

- au moins un, et de préférence chaque, système de collimation comprend un diaphragme aval positionné entre la source lumineuse et le capteur lumineux correspondants du côté du capteur lumineux par rapport à la portion tubulaire, le diaphragme aval étant prévu pour laisser passer une portion centrale des faisceaux lumineux transmis à travers la portion tubulaire en direction du capteur lumineux et pour stopper une portion périphérique des faisceaux lumineux transmis à travers la portion tubulaire ;

- au moins un, et de préférence chaque, système de collimation comprend un filtre amont positionné entre la source lumineuse et le capteur lumineux correspondants du côté de la source lumineuse par rapport à la portion tubulaire, et/ou un filtre aval positionné entre la source lumineuse et le capteur lumineux correspondants du côté du capteur lumineux par rapport à la portion tubulaire, les filtres amont et aval du système de collimation d'un ensemble émetteur-récepteur étant prévus pour filtrer au moins la longueur d'onde d'émission de la source lumineuse de l'autre ensemble émetteur-récepteur ;

- l'appareil est tel que la source lumineuse d'un premier des deux ensembles émetteur-récepteur est configurée pour émettre des faisceaux lumineux à une longueur d'onde comprise entre 780 nm et 840 nm, de préférence comprise entre 800 nm et 820 nm, et de préférence encore égale à 810 nm ; et la source lumineuse d'un deuxième des deux ensembles émetteur-récepteur est configurée pour émettre des faisceaux lumineux à une longueur d'onde comprise entre 1270 nm et 1330 nm, de préférence comprise entre 1290 nm et 1310 nm, et de préférence encore égale à 1300 nm ;

- les sources lumineuses des ensembles émetteur-récepteur sont positionnés du même côté par rapport à la portion tubulaire ;

- l'appareil comprend en outre un système de contrôle des ensembles émetteur-récepteur, le système de contrôle comprenant des moyens de synchronisation des sources lumineuses et/ou des moyens de modification de la puissance émise par les sources lumineuses ;

- les ensembles émetteur-récepteur sont assemblés sur un unique support ayant une rainure destinée à recevoir la portion tubulaire ;

- l'appareil comprend en outre un couvercle prévu pour recouvrir au moins partiellement la rainure, ledit couvercle comprenant une portion de compression destinée à maintenir en position la portion tubulaire positionnée dans la rainure ;

- les sources lumineuses et tous éléments des ensembles émetteur-récepteur prévus pour être du côté de la source lumineuse correspondante par rapport à la portion tubulaire sont assemblés sur un support amont, et les capteurs lumineux et tous éléments des ensembles émetteur-récepteur prévus pour être du côté du capteur lumineux correspondant par rapport à la portion tubulaire sont assemblés sur un support aval distinct du support amont, les supports aval et amont ayant des formes complémentaires prévues pour être couplées de manière à enserrer la portion tubulaire ;

- l'appareil comprend un système de déformation de la portion tubulaire en regard des ensembles émetteur-récepteur, le système de déformation étant prévu pour déformer une section circulaire de la portion tubulaire en une section ellipsoïdale ;

- les sources lumineuses et tous éléments des ensembles émetteur-récepteur prévus pour être du côté de la source lumineuse correspondante par rapport à la portion tubulaire sont positionnés d'un côté d'un grand axe définissant la section ellipsoïdale, et les capteurs lumineux et tous éléments des ensembles émetteur-récepteur prévus pour être du côté du capteur lumineux correspondant par rapport à la portion tubulaire sont positionnés de l'autre côté du grand axe définissant la section ellipsoïdale ;

- la section ellipsoïdale est définie par un grand rayon (Ra) le long du grand axe et par un petit rayon (Rb) le long d'un petit axe perpendiculaire au grand axe, la section ellipsoïdale ayant, dans un état déformé de la portion tubulaire, un petit rayon (Rb) ayant une longueur comprise entre 30% et 70%, et de préférence de l'ordre de 50%, du rayon de la section circulaire de la portion tubulaire en état non-déformé.

On propose en outre un procédé de détermination du taux d'hématocrite et/ou du taux d'hémoglobine d'un liquide en circulation dans une portion tubulaire, comprenant :

- l'émission de faisceaux lumineux en direction de la portion tubulaire avec au moins deux sources lumineuses, chacune des deux sources lumineuses étant configurée pour émettre des faisceaux lumineux selon une longueur d'onde d'émission choisie pour correspondre à un point isobestique de l'hémoglobine ;
- la réception de signaux lumineux transmis à travers la portion tubulaire avec au moins deux capteurs lumineux, chaque capteur lumineux étant associé à l'une des deux sources lumineuses ;
- calcul du taux d'hématocrite et/ou du taux d'hémoglobine du liquide par un traitement des signaux lumineux reçus par les capteurs lumineux ;

caractérisé en ce que la puissance d'émission d'au moins une des sources lumineuses est modifiée au cours de la détermination du taux d'hématocrite et/ou du taux d'hémoglobine en fonction du taux d'hématocrite et/ou respectivement du taux d'hémoglobine calculé pour le liquide.

Des aspects préférés mais non limitatifs de ce procédé, pris seuls ou en combinaison, sont les suivants :

- la puissance d'émission utilisée pour les sources lumineuses est au maximum égale à 100% de la puissance maximum d'émission desdites sources lumineuses, et de préférence comprise entre 10% et 60% de la puissance maximum d'émission desdites sources lumineuses ;
- la puissance d'émission des sources lumineuses est contrôlée de manière indépendante pour chacune des sources lumineuses ;
- la puissance d'émission d'au moins une des sources lumineuses est augmentée à partir d'une valeur seuil du taux d'hématocrite et/ou du taux d'hémoglobine calculé pour le liquide ;
- le procédé est tel que

  ○ la puissance d'émission de la source lumineuse est fixée à une valeur comprise entre 10% et 30%, de préférence égale à 20%, de la puissance maximum d'émission de ladite source lumineuse lorsque le taux d'hématocrite calculé est inférieur à 30% ; et
  ○ la puissance d'émission de la source lumineuse est fixée à une valeur comprise entre 30% et 100%, de préférence égale à 55%, de la puissance maximum d'émission de ladite source lumineuse lorsque le taux d'hématocrite calculé est supérieur ou égal à 30% ;

- la puissance d'émission d'au moins une des sources lumineuses est réglée de sorte que

  ○ la puissance d'émission de ladite source lumineuse est à un premier niveau de puissance pour des valeurs du taux d'hématocrite calculé pour le liquide inférieures à une première valeur seuil ;
  ○ la puissance d'émission de ladite source lumineuse est à un deuxième niveau de puissance pour des valeurs du taux d'hématocrite calculé pour le liquide supérieures ou égales à la première valeur seuil mais inférieure à une deuxième valeur seuil supérieure à la première valeur seuil ; et
  ○ la puissance d'émission de ladite source lumineuse est à un troisième niveau de puissance pour des valeurs du taux d'hématocrite calculé pour le liquide supérieures ou égales à la deuxième valeur seuil ;

- la puissance d'émission d'au moins une des sources lumineuses est réglée de sorte que

  ○ la puissance d'émission de la source lumineuse est fixée à une valeur comprise entre 5% et 15%, de préférence égale à 10%, de la puissance maximum d'émission de ladite source lumineuse lorsque le taux d'hématocrite calculé est inférieur à 20% ;
  ○ la puissance d'émission de la source lumineuse est fixée à une valeur comprise entre 15% et 30%, de préférence égale à 20%, de la puissance maximum d'émission de ladite source lumineuse lorsque le taux d'hématocrite calculé est compris entre 20% et 30% ; et
  ○ la puissance d'émission de la source lumineuse est fixée à une valeur comprise entre 30% et 100%, de préférence égale à 55%, de la puissance maximum d'émission de ladite source lumineuse lorsque le taux d'hématocrite calculé est supérieur ou égal à 30%.

- la puissance d'émission des sources lumineuses est modifiée au cours de la détermination du taux d'hématocrite et/ou du taux d'hémoglobine en fonction de la présence ou non de liquide dans la portion tubulaire et/ou de la nature dudit liquide ;
- les sources lumineuses sont contrôlées pour émettre des faisceaux lumineux de façon concomitante.

DESCRIPTION DES FIGURES

D'autres caractéristiques et avantages de l'invention ressortiront encore de la description qui suit, laquelle est purement illustrative et non limitative et doit être lue en regard des dessins annexés, sur lesquels :

- La figure 1 illustre schématiquement l'agencement et le fonctionnement de l'appareil proposé pour la détermination du taux d'hématocrite et/ou du taux d'hémoglobine d'un liquide en circulation, selon un premier exemple de réalisation.

- La figure 2 illustre schématiquement l'agencement et le fonctionnement de l'appareil proposé pour la détermination du taux d'hématocrite et/ou du taux d'hémoglobine d'un liquide en circulation, selon un deuxième exemple de réalisation.
- La figure 3 illustre schématiquement l'agencement et le fonctionnement de l'appareil proposé pour la détermination du taux d'hématocrite et/ou du taux d'hémoglobine d'un liquide en circulation, selon un troisième exemple de réalisation.
- La figure 4 illustre schématiquement l'agencement et le fonctionnement de l'appareil proposé pour la détermination du taux d'hématocrite et/ou du taux d'hémoglobine d'un liquide en circulation, selon un quatrième exemple de réalisation.
- La figure 5 illustre les ensembles émetteur-récepteur (10 ; 20) de l'appareil proposé, monté sur un support.
- La figure 6 est une vue en perspective de l'appareil proposé pour la détermination du taux d'hématocrite et/ou du taux d'hémoglobine d'un liquide en circulation.
- La figure 7 illustre le montage des systèmes de collimation dans les fûts de montage prévus pour l'assemblage des ensembles émetteur-récepteur.
- La figure 8 illustre le montage des fûts de montage prévus pour l'assemblage des ensembles émetteur-récepteur sur le support.

## DESCRIPTION DETAILLEE DE L'INVENTION

Dans le reste de la description, on parlera de la détermination du taux d'hématocrite d'un liquide en circulation mais les enseignements pourront être appliqués pour d'autres types de paramètres sanguins comme le taux d'hémoglobine par exemple.

### *Appareil pour la détermination du taux d'hématocrite d'un liquide en circulation*

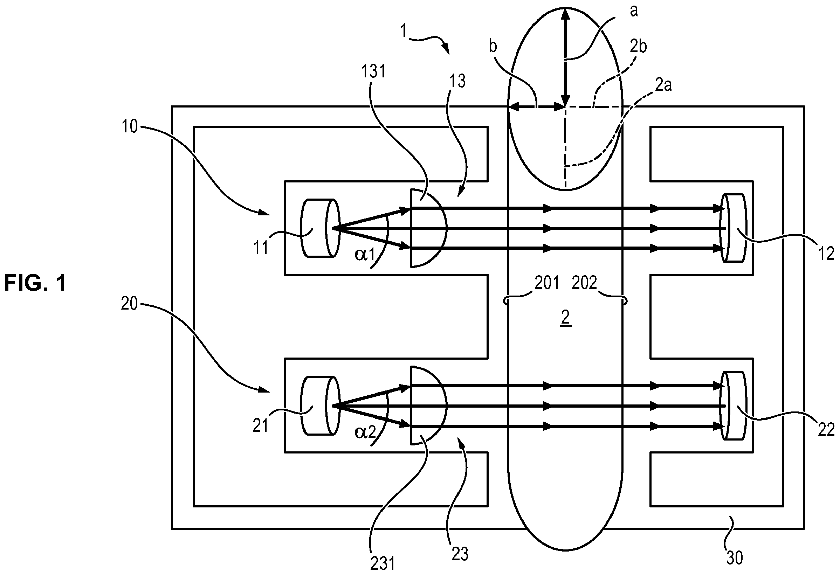

A la figure 1 est représenté de manière schématique l'agencement de l'appareil 1 proposé pour la détermination du taux d'hématocrite d'un liquide en circulation dans une portion tubulaire 2.

L'appareil 1 de détermination du taux d'hématocrite proposé peut être utilisé pour déterminer le taux d'hématocrite de tout type de liquide mais il est particulièrement adapté pour déterminer le taux d'hématocrite de liquide hémorragique tel que du sang humain, en circulation dans une tubulure, par exemple un tube souple utilisé de manière standard en milieu hospitalier.

Comme il sera détaillé plus loin, l'appareil 1 proposé permet de déterminer le taux d'hématocrite d'un liquide de manière non-invasive, c'est-à-dire sans avoir à intervenir sur le liquide en tant que tel, qui peut donc continuer à circuler librement dans la tubulure.

L'appareil 1 proposé comprend au moins deux ensembles émetteur-récepteur (10 ; 20), chaque ensemble émetteur-récepteur (10 ; 20) comprenant une source lumineuse (11 ; 21) et un capteur lumineux (12 ; 22). Ces deux ensembles émetteur-récepteur (10 ; 20) sont utilisés pour déterminer le taux d'hématocrite du liquide en circulation dans la tubulure, les faisceaux lumineux traversant le liquide à analyser étant utilisés pour calculer le taux d'hématocrite du liquide. Le fait d'avoir deux ensembles émetteur-récepteur (10 ; 20) permet d'augmenter la fiabilité de l'appareil 1 puisque les mesures des deux capteur lumineux (12 ; 22) peuvent être corrélées. En outre, cela permet d'avoir une redondance qui peut être avantageuse en cas de défaillance de l'un des deux ensembles émetteur-récepteur (10 ; 20).

La source lumineuse (11 ; 21) de chacun des deux ensembles émetteur-récepteur (10 ; 20) est configurée pour émettre des faisceaux lumineux selon une longueur d'onde d'émission choisie pour correspondre à un point isobestique de l'hémoglobine. Il est entendu qu'un point isobestique correspond à une valeur de longueur d'onde à laquelle l'absorbance totale d'un échantillon reste constante durant une réaction chimique ou un possible changement d'état de cet échantillon. Plus précisément, un point isobestique est une longueur d'onde ($\lambda_{iso}$) à laquelle l'absorbance totale d'un chromophore reste constante peu importe l'état dans lequel il se trouve. En ce point précis plusieurs chromophores possèdent le même coefficient d'extinction molaire ($\alpha(\lambda_{iso})$).

Il existe pour l'hémoglobine plusieurs points isobestiques.

Par exemple, l'oxyhémoglobine et la désoxyhémoglobine possèdent des points isobestiques à 550 nm, 570 nm et proche de 810 nm de longueur d'onde. A ces longueurs d'ondes ($\lambda_{iso}$), on peut donc obtenir une mesure liée au volume total d'hémoglobine, puisque l'absorption de la lumière à cette longueur d'onde est indépendante de l'état oxygéné ou réduit dans lequel se trouve l'hémoglobine.

En outre, une longueur d'onde proche de 1300 nm correspond à un autre point isobestique de l'hémoglobine.

Les longueurs d'onde supérieures à 1400 nm sont aussi généralement isobestiques de l'hémoglobine, en particulier les longueurs d'onde comprises entre 1400 nm et 2200 nm. Un avantage de ces longueurs d'onde spécifiques réside dans le fait que l'absorption de la lumière à ces longueurs d'onde est sensiblement la même dans l'eau et dans le plasma. Ainsi, le taux d'hématocrite déterminé à ces longueurs d'onde sera le même quelle que soit la matrice portant les globules rouges, que cette matrice soit majoritairement composée de plasma ou qu'elle soit majoritairement composée d'eau. Ceci est particulièrement vrai pour les longueurs d'onde comprises entre 1400 nm et 1700 nm et comprises entre 1900 nm et 2200 nm. De telles longueurs d'onde sont donc particulièrement avantageuses lorsque le liquide hémorragique pour lequel le taux d'hématocrite est à déterminé est dilué plus ou moins fortement avec une solution aqueuse, telle qu'une solution saline par exemple.

Ainsi, l'une des sources lumineuses (11 ; 21) des ensembles émetteur-récepteur (10 ; 20) peut par exemple être configurée pour émettre des faisceaux lumineux à une longueur d'onde comprise entre 780 nm et 840 nm, de préférence une longueur d'onde comprise entre 800 nm et 820 nm, et de préférence encore une longueur d'onde égale à 810 nm.

L'une des sources lumineuses (11 ; 21) des ensembles émetteur-récepteur (10 ; 20) peut par exemple être configurée pour émettre des faisceaux lumineux à une longueur d'onde comprise entre 1270 nm et 1330 nm, de préférence une longueur d'onde comprise entre 1290 nm et 1310 nm, et de préférence encore une longueur d'onde égale à 1300 nm.

L'une des sources lumineuses (11 ; 21) des ensembles émetteur-récepteur (10 ; 20) peut par exemple être configurée pour émettre des faisceaux lumineux à une longueur d'onde comprise entre 1450 nm et 1550 nm, de préférence une longueur d'onde comprise entre 1490 nm et 1510 nm, et de préférence encore une longueur d'onde égale à 1500 nm.

L'une des sources lumineuses (11 ; 21) des ensembles émetteur-récepteur (10 ; 20) peut par exemple être configurée pour émettre des faisceaux lumineux à une longueur d'onde comprise entre 530 nm et 620 nm, de préférence une longueur d'onde comprise entre 550 nm et 600 nm, et de préférence encore une longueur d'onde égale à 550 nm, à 570 nm ou à 590 nm.

Les sources lumineuses respectives (11 ; 21) des deux ensembles émetteur-récepteur (10 ; 20) peuvent être configurées pour émettre des faisceaux lumineux à deux longueurs d'onde d'émission identiques, mais il est avantageux que les deux sources lumineuses (11 ; 21) soient configurées pour émettre des faisceaux lumineux à deux longueurs d'onde d'émission différentes. Outre l'avantage mentionné plus haut dû à la redondance fonctionnelle des deux ensembles émetteur-récepteur (10 ; 20), l'utilisation de deux sources lumineuses fonctionnant à des longueurs d'onde différentes permet une meilleure corrélation des mesures en vue du calcul du taux d'hématocrite du liquide en circulation dans la portion tubulaire 2.

Selon un exemple particulier, l'une des sources lumineuses (11 ; 21) des ensembles émetteur-récepteur (10 ; 20) est configurée pour émettre des faisceaux lumineux à une longueur d'onde comprise entre 780 nm et 840 nm, de préférence une longueur d'onde comprise entre 800 nm et 820 nm, et de préférence encore une longueur d'onde égale à 810 nm, tandis que l'autre source lumineuse (11 ; 21) est configurée pour émettre des faisceaux lumineux à une longueur d'onde comprise entre 1270 nm et 1330 nm, de préférence une longueur d'onde comprise entre 1290 nm et 1310 nm, et de préférence encore une longueur d'onde égale à 1300 nm. Il peut par exemple être utilisé des diodes électroluminescentes (abrégé en anglais en LED pour « light-emitting diode »), comme celle proposée par la société « THORLABS » sous la référence LED810L (pour une source lumineuse à 810 nm) et celle proposée par la société « MARKTECH Optoelectronics » sous la référence MTE1300NN1-WRC (pour une source lumineuse à 1300 nm).

Comme illustré sur la figure 1, la source lumineuse (11 ; 21) et le capteur lumineux (12 ; 22) de chaque ensemble émetteur-récepteur (10 ; 20) sont prévus pour être agencés de part et d'autre de la portion tubulaire 2 au niveau d'une zone de circulation du liquide, qui forme une zone de détection des ensembles émetteur-récepteur (10 ; 20). Un tel agencement va permettre une mesure en transmission, c'est-à-dire que les faisceaux lumineux issus de chacune des sources lumineuses (11 ; 21) sont destinés à traverser le liquide en circulation dans la portion tubulaire 2 avant d'atteindre le capteurs lumineux (12 ; 22) de l'ensemble émetteur-récepteur (10 ; 20) correspondant. Plus précisément, chaque faisceau lumineux en provenance des sources lumineuses (11 ; 21) traverse une première paroi de la portion tubulaire 2, appelée paroi d'entrée 201, puis transverse le liquide circulant dans la portion tubulaire 2, puis traverse une deuxième paroi de la portion tubulaire 2, opposée à la paroi d'entrée 201 et appelée paroi de sortie 202.

Selon un mode de réalisation avantageux, les sources lumineuses (11 ; 21) des ensembles émetteur-récepteur (10 ; 20) sont positionnées du même côté par rapport à la portion tubulaire 2. Cela permet notamment de faciliter le montage des différents éléments formant l'appareil 1 de détermination du taux d'hématocrite et améliore la compacité de l'appareil 1 puisque les éléments semblables et donc de même taille sont placés du même côté.

Avantageusement, chaque ensemble émetteur-récepteur (10 ; 20) comprend en outre un système de collimation (13 ; 23) prévu pour une collimation du faisceau lumineux émis depuis la source lumineuse (11 ; 21) correspondante en direction du capteur lumineux (12 ; 22) associé.

Plus précisément, de tels systèmes de collimation (13 ; 23) sont configurés pour une collimation à l'infini des faisceaux lumineux en provenance des sources lumineuses (11 ; 21) en direction de la portion tubulaire 2.

Lorsque les faisceaux lumineux en provenance des sources lumineuses (11 ; 21) traversent la portion tubulaire 2 dans laquelle circule le liquide, le chemin optique de ces faisceaux lumineux est modifié par la traversée de la paroi d'entrée 201 de la portion tubulaire 2, par la traversée du liquide circulant dans la portion tubulaire 2, puis par la traversée de la paroi d'entrée 202 de la portion tubulaire 2. Le fait de collimater à l'infini les faisceaux lumineux en provenance des sources lumineuses (11 ; 21) permet de faire converger les faisceaux lumineux en direction des capteurs lumineux (12 ; 22) des ensembles émetteur-récepteur (10 ; 20) quelle que soit la forme de la portion tubulaire, en particulier si cette portion tubulaire 2 est non déformée avec une section circulaire ou si cette portion tubulaire 2 est déformée avec une section ellipsoïdale.

Chaque système de collimation (13 ; 23), ou au moins l'un des deux, peut par exemple comprendre un ensemble de lentille(s) amont (131 ; 231) ayant un plan focal et étant positionné entre la source lumineuse (11 ; 21) et le capteur lumineux (12 ; 22) correspondants du côté de la source lumineuse (11 ; 21) par rapport à la portion tubulaire 2. Un tel ensemble de lentille(s) amont (131 ; 231) peut être constitué d'une seule lentille ayant un plan focal unique ou d'une pluralité de lentilles dont l'assemblage permet de définir un plan focal global.

Selon un exemple de réalisation, la source lumineuse (11 ; 21) peut être positionnée à plus ou moins 10 mm du plan focal de l'ensemble de lentille(s) amont, et de préférence dans le plan focal de l'ensemble de lentille(s) amont.

Par ailleurs, chaque système de collimation (13 ; 23), ou au moins l'un des deux, peut comprendre un ensemble de lentille(s) aval ayant un plan focal et étant positionné entre la source lumineuse (11 ; 21) et le capteur lumineux (12 ; 22) correspondants du côté du capteur lumineux (12 ; 22) par rapport à la portion tubulaire 2. Un tel ensemble de lentille(s) aval peut être constitué d'une seule lentille ayant un plan focal unique ou d'une pluralité de lentilles dont l'assemblage permet de définir un plan focal global.

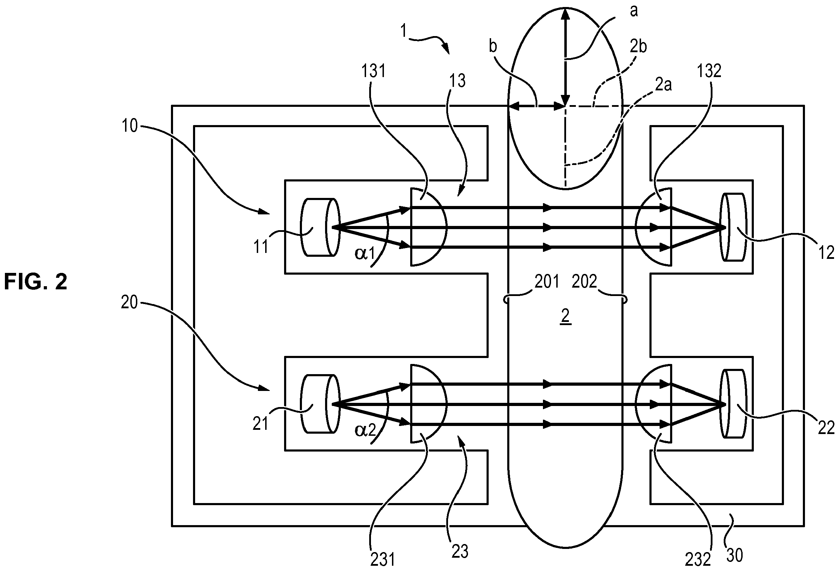

Selon un exemple de réalisation illustré à la figure 2, le capteur lumineux (12 ; 22) peut par exemple être positionné à plus ou moins 10 mm du plan focal de l'ensemble de lentille(s) aval (132 ; 232), et de préférence dans plan focal de l'ensemble de lentille(s) aval (132 ; 232). Ainsi, lorsque les faisceaux lumineux en sortie de la paroi de sortie 202 de la portion tubulaire 2 sont collimatés sensiblement à l'infini, cet ensemble de lentille(s) aval permet de faire converger ces faisceaux lumineux sur le capteur lumineux (12 ; 22) correspondant.

Selon un autre exemple de réalisation, l'ensemble de lentille(s) aval est positionné de sorte que les faisceaux lumineux en sortie de la paroi de sortie 202 de la portion tubulaire 2 convergent à plus ou moins 10 mm du plan focal de l'ensemble de lentille(s) aval, et de préférence dans le plan focal de cet ensemble de lentille(s) aval. Ainsi, cet ensemble de lentille(s) aval permet de collimater à l'infini les faisceaux lumineux en direction du capteur lumineux (12 ; 22) correspondant.

Il est à noter que l'ensemble de lentille(s) amont et/ou l'ensemble de lentille(s) aval pourraient être montés dans l'appareil 1 de manière à pouvoir être translatés selon l'axe optique général, par exemple de manière automatisé, de sorte à pouvoir faire varier leur positionnement selon les dimensions et la déformation de la portion tubulaire 2 dans laquelle circule le liquide dont le taux d'hématocrite est à déterminer.

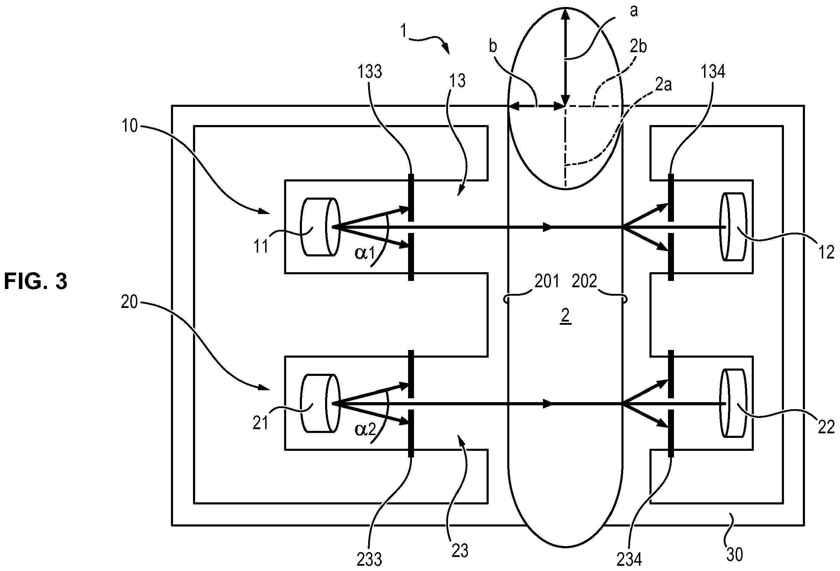

Par ailleurs, comme illustré à la figure 3, chaque système de collimation (13 ; 23), ou au moins l'un des deux, peut comprendre un diaphragme amont (133 ; 233) positionné entre la source lumineuse (11 ; 21) et le capteur lumineux (12 ; 22) correspondants du côté de la source lumineuse (11 ; 21) par rapport à la portion tubulaire 2. Un tel diaphragme amont (133 ; 233) est configuré et agencé dans l'appareil 1 pour laisser passer une portion centrale des faisceaux lumineux émis par la source lumineuse (11 ; 21) en direction du capteur lumineux (12 ; 22) et pour stopper une portion périphérique des faisceaux lumineux émis par la source lumineuse (11 ; 21).

En complément ou en alternative, comme illustré à la figure 3, chaque système de collimation (13 ; 23), ou au moins l'un des deux, peut comprendre un diaphragme aval (134 ; 234) positionné entre la source lumineuse (11 ; 21) et le capteur lumineux (12 ; 22) correspondants du côté du capteur lumineux (12 ; 22) par rapport à la portion tubulaire 2. Un tel diaphragme aval (134 ; 234) est configuré et agencé dans l'appareil 1 pour laisser passer une portion centrale des faisceaux lumineux transmis à travers la portion tubulaire 2 en direction du capteur lumineux (12 ; 22) et pour stopper une portion périphérique des faisceaux lumineux transmis à travers la portion tubulaire 2.

L'utilisation d'un diaphragme amont (133 ; 233) et/ou d'un diaphragme aval (134 ; 234) est particulièrement avantageux puisque cela permet de s'affranchir des faisceaux lumineux qui bruitent la réception des capteurs lumineux (12 ; 22) et viennent donc perturber les mesures de l'appareil 1. De tels diaphragmes permettent par exemple de réduire les bruits causés par les faisceaux lumineux réfléchis, diffractés ou diffusés par la portion tubulaire 2. En effet, le diaphragme amont (133 ; 233) permet de sélectionner et centrer le faisceau lumineux émis depuis la source lumineuse (11 ; 21) sur la portion tubulaire 2 afin de diminuer au maximum la diffusion et réflexion de celle-ci. Le diaphragme aval (134 ; 234) permet quant à lui de raffiner encore le signal reçu par le capteur lumineux (12 ; 22) puisqu'il ne laisse passer que les faisceaux centraux transmis par la portion tubulaire 2 tout en coupant les faisceaux parasites tels que les faisceaux lumineux réfléchis, diffractés ou diffusés par la portion tubulaire 2. Un autre avantage de l'utilisation de diaphragme(s) est qu'ils permettent d'améliorer le niveau de réception par les capteurs lumineux (12 ; 22) sans avoir à augmenter la puissance des sources lumineuses (11 ; 21) ce qui permet d'augmenter la durée de vie des composant de l'appareil 1. Il est à noter que l'utilisation de diaphragmes, notamment du diaphragme amont, sera d'autant plus privilégiée que le cône d'émission des sources lumineuses (11 ; 21) sera resserré, c'est-à-dire que les angles ($\alpha$1 ; $\alpha$2) des cônes d'émission des sources lumineuses (11 ; 21) sera faible, afin qu'une majeure partie du faisceau lumineux issue des sources lumineuses (11 ; 21) soit concentrée en coupant uniquement les fasceaux lumineux périphériques parasites.

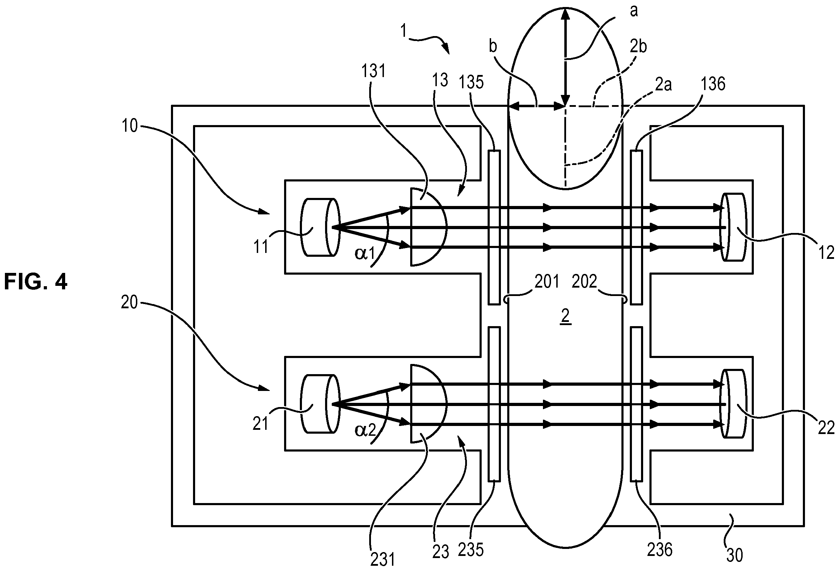

En complément ou en alternative, et comme illustré à la figure 4, chaque système de collimation (13 ; 23), ou au moins l'un des deux, peut comprendre un filtre amont (135 ; 235) positionné entre la source lumineuse (11 ; 21) et le capteur lumineux (12 ; 22) correspondants du côté du capteur lumineux (12 ; 22) par rapport à la portion tubulaire 2. Un tel filtre aval (136 ; 236) du système de collimation (13 ; 23) d'un ensemble émetteur-récepteur (10 ; 20) est prévu pourfiltrer au moins la longueur d'onde d'émission de la source lumineuse (11 ; 21) de l'autre ensemble émetteur-récepteur (10 ; 20). De manière préférée, le filtre aval (136 ; 236) du système de collimation (13 ; 23) d'un ensemble émetteur-récepteur (10 ; 20) est prévu pour bloquer tous les faisceaux lumineux n'étant pas à la longueur d'onde d'intérêt, c'est-à-dire la longueur d'onde d'émission de la source lumineuse (11 ; 21) correspondante.

L'utilisation d'un filtre amont (135 ; 235) et/ou d'un filtre aval (136 ; 236) est particulièrement avantageux en ce que cela permet de limiter le faisceau lumineux reçu par un capteur lumineux (12 ; 22) particulier aux seuls faisceaux lumineux issus de la source lumineuse (11 ; 21) correspondante, tout en évitant une perturbation par des faisceaux lumineux parasites issus de l'autre source lumineuse (11 ; 21), voire des faisceaux parasites issues de la lumière ambiante.

Comme il a été indiqué plus haut, il est préférable que les cônes d'émission des sources lumineuses (11 ; 21) définis par les angles (α1 ; α2) soient aussi resserrés que possibles pour que l'intensité du faisceau lumineux centré sur la portion tubulaire 2 soit la plus forte possible sans avoir à utiliser une puissance d'émission trop forte pour les sources lumineuses (11 ; 21) pour ne pas réduire la durée de vie des éléments formant l'appareil 1.

Ainsi, les angles (α1 ; α2) des cônes d'émission des sources lumineuses (11 ; 21) est de préférence compris entre 1° et 25°, et de manière préférée entre 5° et 20°, et de préférence encore entre 10° et 15°. Il est à noter que l'angle (α1 ; α2) de cône d'émission peut dépendre de la longueur d'onde d'émission utilisée pour la source lumineuse (11 ; 21).

Pour une source lumineuse (11 ; 21) émettant à une longueur d'onde proche de 810 nm, l'angle (α1 ; α2) de cône d'émission peut par exemple être de l'ordre de 13° ($\pm$1°).

Pour une source lumineuse (11 ; 21) émettant à une longueur d'onde proche de 1300 nm, l'angle (α1 ; α2) de cône d'émission peut par exemple être de l'ordre de 15° ($\pm$1°).

Comme il a été précisé plus haut, l'appareil 1 de détermination du taux d'hématocrite proposé est prévu pour être positionné autour d'une portion tubulaire existante, par exemple une tubulure ou tube souple utilisé dans un système de traitement de liquide hémorragique, de sorte à permettre une détermination du taux d'hématocrite de manière non invasive.

A cette fin, l'appareil 1 comprend un ensemble support 30 sur lequel sont montés les éléments formant l'appareil 1, en particulier les ensembles émetteur-récepteur (10 ; 20). Comme indiqué précédemment, l'ensemble support 30 est de préférence prévu pour être positionné autour de la portion tubulaire.

Un tel ensemble support 30 peut par exemple comprendre un unique support 31 tel qu'illustré aux figures 1 à 6, ce support 31 ayant une rainure 32 destinée à recevoir la portion tubulaire 2. Les sources lumineuses (11 ; 21) et les capteurs lumineux (12 ; 22) sont alors agencés sur le support 31 de part et d'autre de la rainure 32.

L'ensemble support peut en outre comprendre un couvercle 33 prévu pour recouvrir au moins partiellement la rainure 32 du support 31. Un tel couvercle 33 est prévu pour empêcher le retrait de la portion tubulaire 2 qui serait insérée dans la rainure 32, ayant ainsi une fonction de verrou.

Selon un mode de réalisation avantageux, le couvercle 33 comprend une portion de compression 331 destinée à mettre en compression la portion tubulaire 2 positionnée dans la rainure 32. Cette portion de compression 331 est adaptée pour au moins maintenir en position la portion tubulaire 2 dans la rainure 32 du support 31 de l'appareil 1.

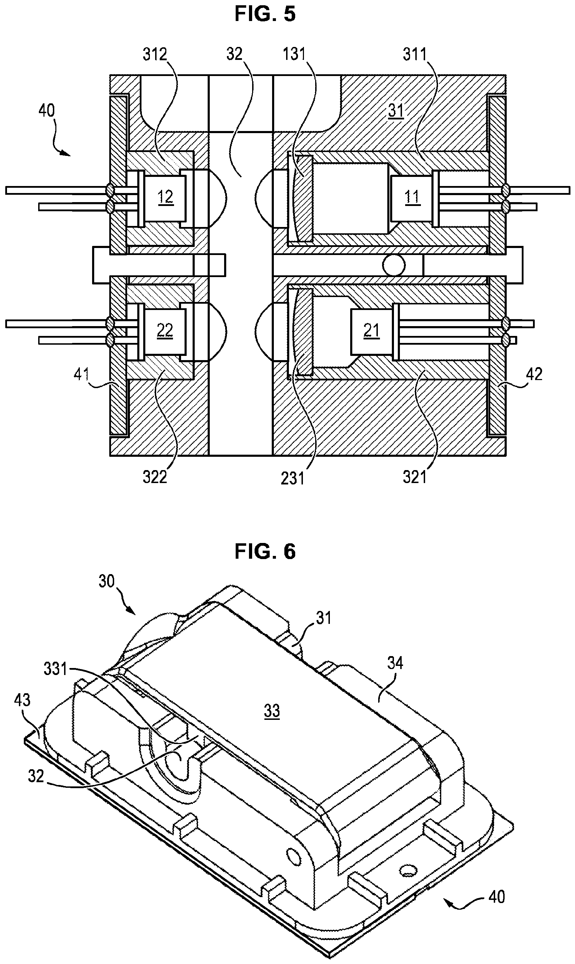

Selon le mode de réalisation illustré à la figure 6, l'ensemble support 30 comprend une enveloppe 34 destinée à recouvrir le support 30, notamment pour protéger les éléments de l'appareil 1. Une telle enveloppe 34 forme le boitier externe de l'appareil 1.

Un tel couvercle 33 peut par exemple être monté de manière articulée par rapport au support 31. Le couvercle 33 est par exemple assemblé sur l'enveloppe 34 de manière articulée et agencée de manière à être en regard de la rainure 32 du support 31.

De préférence, le couvercle 33 et/ou l'enveloppe 34 ont des surfaces externes permettant de protéger les ensembles émetteur-récepteur (10 ; 20) de perturbations externes, notamment de la lumière ambiante.

Le couvercle 33 et/ou l'enveloppe 34 ont également de préférence des surfaces externes empêchant la réflexion des rayons lumineux dus aux sources lumineuses (11 ; 21) et non dirigés vers les capteurs lumineux (12 ; 22), tels que par exemple tous rayons diffusés, diffractés, réfléchis. De préférence, les surfaces externes du couvercle 33 et/ou de l'enveloppe 34 sont prévues pour absorber ces rayons lumineux.

Le couvercle 33 et/ou l'enveloppe 34 peuvent par exemple être formés dans un matériau totalement opaque.

Le couvercle 33 et/ou l'enveloppe 34 sont en outre de préférence prévus pour garantir une étanchéité de l'appareil 1, en particulier une étanchéité aux liquides afin de protéger tous les éléments sensibles de l'appareil 1, notamment les composants électroniques.

Le fait d'avoir un support 31 unique pour l'appareil 1 permet un montage précis et un maintien en position des éléments formant les ensembles émetteur-récepteur (10 ; 20). Un tel mode de réalisation est particulièrement avantageux puisqu'il permet notamment de se rapprocher au maximum des conditions optiques optimales pour les faisceaux lumineux, en particulier s'agissant de leur centrage par rapport à la portion tubulaire 2.

Chaque élément formant les ensembles émetteur-récepteur (10 ; 20) peut être monté individuellement sur le support 31 afin de former l'appareil 1. L'unicité du support 31 permet de garantir un maintien en position des éléments les uns par rapport aux autres mais le montage en tant que tel peut s'avérer difficile. Pour faciliter le montage des éléments formant les ensembles émetteur-récepteur (10 ; 20) tout en garantissant un positionnement précis, il est proposé d'utiliser des fûts de montage (311 ; 321 ; 312 ; 322) dans lesquels les élément formant les ensembles émetteur-récepteur (10 ; 20) sont prémontés, lesquels fûts de montage (311 ; 321 ; 312 ; 322) étant ensuite insérés dans des cavités de montage ménagées dans le support 31, ces cavités de montage ayant une forme complémentaire des fûts de montage (311 ; 321 ; 312 ; 322), permettant par exemple une insertion en force des fûts de montage (311 ; 321 ; 312 ; 322) dans ces cavités de montage.

Dans chaque fût de montage (311 ; 321 ; 312 ; 322) sont ménagées une ou plusieurs cavités de réception des éléments formant les ensembles émetteur-récepteur (10 ; 20), chaque cavité étant dimensionnée pour recevoir l'élément spécifique à positionner.

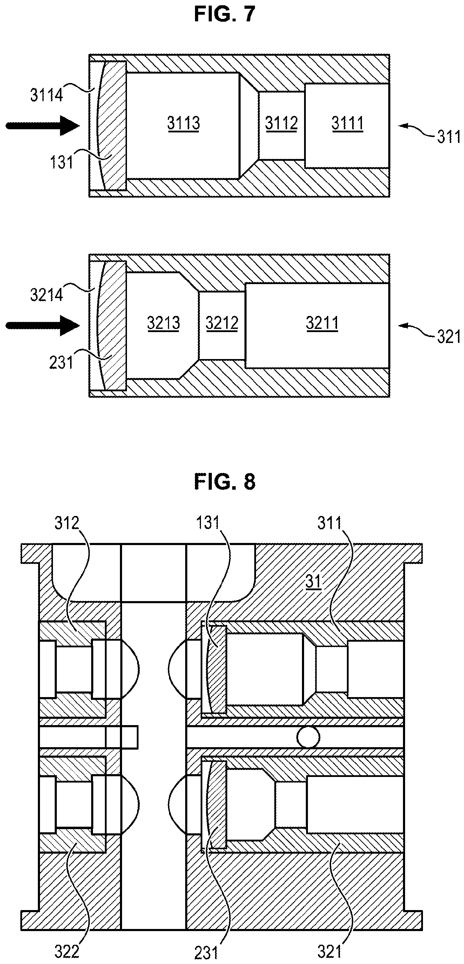

A la figure 7 sont représentés des exemples de réalisation de fûts de montage (311 ; 312) pour les éléments formant le système de collimation (13 ; 23) de l'appareil 1 selon l'exemple de la figure 1, et des sources lumineuses (11 ; 21). Chaque fût de montage (311 ; 312) a une forme sensiblement allongée, de préférence cylindrique, et comprend plusieurs cavités (3111, 3112, 3113, 3114 ; 3121, 3122, 3123, 3124) reliées les unes aux autres de sorte à former une ouverture traversante à travers le fût de montage (311 ; 312). De préférence, deux cavités (3111, 3112, 3113, 3114 ; 3121, 3122, 3123, 3124) adjacentes ont des sections de taille et/ou de forme différentes de sorte que la coopération de cavités (3111, 3112, 3113, 3114 ; 3121, 3122, 3123, 3124) adjacentes permet de former un espace de réception d'un élément formant le système de collimation (13 ; 23) ou la source lumineuse (11 ; 21). Les cavités (3111, 3112, 3113, 3114 ; 3121, 3122, 3123, 3124) sont donc formées dans le fût de montage (311 ; 312) afin de permettre un positionnement précis des éléments formant les ensembles émetteur-récepteur (10 ; 20).

Selon le mode de réalisation illustré à la figure 7, le système de collimation (13 ; 23) comprend une lentille (131 ; 231) qui peut être insérée dans la cavité (3114; 3214) d'extrémité et venir en butée contre l'épaulement formé par la cavité (3113; 3213) adjacente.

La cavité (3113 ; 3213) sur laquelle la lentille (131 ; 231) vient en butée a une longueur correspondant à la distance souhaitée entre la lentille (131 ; 231) et la source lumineuse (11 ; 21) correspondante. De préférence, cette longueur est choisie pour que la source lumineuse (11 ; 21) soit dans le plan focal de la lentille (131 ; 231). Cette cavité (3113 ; 3213) a donc une fonction de maintien à distance.

La source lumineuse (11 ; 21) est quant à elle destinée à être insérée depuis l'autre cavité (3111 ; 3211) d'extrémité jusque dans la cavité (3112 ; 3212) adjacente, cette cavité (3112 ; 3212) étant également adjacente à la cavité (3113 ; 3213) de maintien à distance. La source lumineuse (11 ; 21) peut par exemple comprendre une protubérance venant en butée contre un épaulement formé entre la cavité (3112 ; 3212) de réception de la source lumineuse (11 ; 21) et la cavité (3111 ; 3211) d'extrémité.

Une fois que la lentille (131 ; 231) est prémontée dans le fût de montage (311 ; 312), il est possible d'insérer ce fût de montage (311 ; 312) dans la cavité de montage prévue à cet effet dans le support 30. La figure 8 illustre cette insertion des fûts de montage (311 ; 321 ; 312 ; 322) dans les cavités de montage prévues à cet effet dans le support 31.

Selon l'exemple illustré à la figure 8, les sources lumineuses (11 ; 21) et les capteurs lumineux (12 ; 22) sont montés dans les cavités ménagées dans les fûts de montage (311 ; 321 ; 312 ; 322) une fois que ces fûts de montage (311 ; 321 ; 312 ; 322) ont été insérés dans le support 30. Il peut toutefois être prévu de prémonter les sources lumineuses (11 ; 21) et les capteurs lumineux (12 ; 22) dans les cavités ménagées dans les fûts de montage (311 ; 321 ; 312 ; 322) avant que ces fûts de montage (311 ; 321 ; 312 ; 322) ne soient insérés dans le support 31.

Selon un agencement alternatif (non représenté), l'ensemble support 30 comprend deux supports destinés à être assemblés l'un avec l'autre en entourant la portion tubulaire 2.

Il peut ainsi être prévu un support amont sur lequel sont agencés les sources lumineuses (11 ; 21) et tous éléments des ensembles émetteur-récepteur (10 ; 20) prévus pour être du côté de la source lumineuse (11 ; 21) correspondante par rapport à la portion tubulaire 2.

Un support aval distinct du support amont est également prévu, sur lequel sont agencés les capteurs lumineux (12 ; 22) et tous éléments des ensembles émetteur-récepteur (10 ; 20) prévus pour être du côté du capteur lumineux (12 ; 22) correspondant par rapport à la portion tubulaire 2.

De préférence, les supports aval et amont ont des formes complémentaires prévues pour être couplées de manière à enserrer la portion tubulaire 2.

L'une des caractéristiques importantes de l'appareil 1 proposé est que le flux du liquide en circulation dans la portion tubulaire 2 n'est pas ou peu modifié de manière à ne pas avoir un impact négatif sur ce liquide. Par exemple, pour un liquide hémorragique tel que du sang, une modification trop importante du flux dû par exemple à une constriction substantielle de la portion tubulaire 2 au niveau de la zone de détection de l'appareil 1 pourrait créer une hémolyse, ce qui est à éviter pour un traitement efficace du liquide hémorragique. En particulier, il n'est pas désiré que la portion tubulaire 2 au niveau de la zone de détection soit aplatie de manière à ce que la paroi d'entrée 201 et la paroi de sortie 202 soient sensiblement parallèles l'une par rapport à l'autre puisque cela créerait une hémolyse trop importante pour le traitement du liquide hémorragique. Ainsi les ensembles émetteur-récepteur (10 ; 20) sont de préférence agencés dans l'appareil 1 pour une mesure en transmission à travers des parois incurvées de la portion tubulaire 2, c'est-à-dire des parois courbes.

La façon la plus simple d'éviter un risque d'hémolyse est de ne pas déformer la portion tubulaire 2. Les courbures prononcées de la section circulaire de la portion tubulaire 2 peuvent toutefois venir perturber la transmission des faisceaux lumineux des sources lumineuses (11 ; 21) vers les capteurs lumineux (12 ; 22). Ainsi, il peut être envisagé de déformer légèrement la portion tubulaire 2, de façon contrôlée (par exemple avec un taux de compression de l'ordre de 2%), de manière à ne pas ou peu perturber le flux du liquide dans la portion tubulaire 2 tout en réduisant les courbures de la portion tubulaire 2 pour réduire leur effet sur l'orientation des faisceaux lumineux traversant la portion tubulaire 2 et ainsi augmenter la précision de mesure de l'appareil 1. Notons ici que l'agencement spécifique de l'appareil 1 et notamment l'utilisation de systèmes de collimation (13 ; 23) dans les ensembles émetteur-récepteur (10 ; 20) permet d'avoir une détection fiable y compris quand la portion tubulaire 2 présente une certaine courbure au niveau de la zone de détection. C'est pourquoi il n'est pas nécessaire d'avoir un aplatissement de la portion tubulaire 2 au niveau de cette zone de détection.

De préférence, l'appareil et la méthode proposés sont prévus pour une détermination du taux d'hématocrite et/ou du taux d'hémoglobine du liquide en circulation sans déformation de la portion tubulaire.

Quel que soit l'ensemble support 30 utilisé pour l'appareil 1, il peut toutefois en outre être prévu un système de déformation de la portion tubulaire 2 positionnée en regard des ensembles émetteur-récepteur (10 ; 20).

De préférence, la portion tubulaire au niveau de laquelle la mesure en transmission est effectuée conserve une certaine courbure, et n'est donc pas aplatie.

Selon un mode de réalisation préféré, le système de déformation est prévu pour déformer la section circulaire de la portion tubulaire en une section ellipsoïdale. A cette fin, le système de déformation peut par exemple utiliser la coopération du couvercle 33, et plus spécifiquement de la portion de compression 331, avec la forme de la rainure 32. La rainure 32 peut en effet avoir une section de forme sensiblement ellipsoïdale et la portion de compression 331 est prévue pour venir comprimer la portion tubulaire 2 de sorte qu'elle se déforme et vienne sensiblement épouser la forme de la rainure 32.

La section ellipsoïdale de la portion tubulaire 2 déformée est définie par un grand axe 2a et un petit axe 2b perpendiculaire au grand axe. De préférence, la déformation est telle que les sources lumineuses (11 ; 21) et tous éléments des ensembles émetteur-récepteur (10 ; 20) prévus pour être du côté de la source lumineuse (11 ; 21) correspondante par rapport à la portion tubulaire 2 sont positionnés d'un côté du grand axe 2a, et les capteurs lumineux (12 ; 22) et tous éléments des ensembles émetteur-récepteur (10 ; 20) prévus pour être du côté du capteur lumineux (12 ; 22) correspondant par rapport à la portion tubulaire 2 sont positionnés de l'autre côté du grand axe 2a.

La section ellipsoïdale de la portion tubulaire 2 déformée est en outre définie par un grand rayon (Ra) le long du grand axe 2a et par un petit rayon (Rb) le long du petit axe 2b, la section ellipsoïdale ayant, dans un état déformé de la portion tubulaire, un petit rayon (Rb) ayant une longueur comprise entre 30% et 70%, et de préférence de l'ordre de 50%, du rayon de la section circulaire de la portion tubulaire 2 en état non-déformé.

Les ensembles émetteur-récepteur (10 ; 20) sont prévus pour être couplés à une unité centrale de traitement permettant de les piloter, aussi bien en émission qu'en réception, mais permettant aussi de traiter les informations des ensembles émetteur-récepteur (10 ; 20).

L'appareil 1 peut par exemple comprendre un système de contrôle relié à l'unité centrale de traitement et configuré pour commander les sources lumineuses (11 ; 21) des ensembles émetteur-récepteur (10 ; 20).

L'appareil 1 peut en outre comprendre un système de traitement relié à l'unité centrale de traitement et configuré pour récupérer et traiter les signaux issus des capteurs lumineux (12 ; 22) des ensembles émetteur-récepteur (10 ; 20), afin notamment de déterminer le taux d'hématocrite du liquide en circulation.

Les sources lumineuses (11 ; 21) et les capteurs lumineux (12 ; 22) des ensembles émetteur-récepteur (10 ; 20) sont ainsi de préférence reliés à un circuit électronique 40 permettant au système de contrôle de commander les sources lumineuses (11 ; 21) d'une part, et au système de traitement de récupérer les signaux reçus par les capteurs lumineux (12 ; 22) d'autre part.

Selon l'exemple illustré aux figures 5, 7 et 8, le circuit électronique 40 comprend une première carte électronique 41 destiné à être connectée aux sources lumineuses (11 ; 21) et une deuxième carte électronique 42 destinée à être connectée aux capteurs lumineux (12 ; 22). Chacune de ces première et deuxième cartes électroniques (41 ; 42) sont de préférence couplées aux sources lumineuses (11 ; 21) et aux capteurs lumineux (12 ; 22) respectivement une fois que ceux-ci ont été montés dans le support 31.

Selon ce mode de réalisation, le circuit électronique 40 comprend en outre une troisième carte électronique 43 reliant les première et deuxième cartes électroniques (41 ; 42). Cette troisième carte électronique 43 peut en outre former une paroi de l'appareil 1 formant avec l'enveloppe 34 le boitier externe de l'appareil 1.

Il pourrait être envisagé de contrôler les sources lumineuses (11 ; 21) pour qu'elles émettent en alternance l'une de l'autre, de manière notamment à réduire les possibles interférences entre les deux ensembles émetteur-récepteur (10 ; 20). La configuration spécifique de l'appareil 1 proposé permet toutefois de ne pas nécessiter cette alternance d'émission puisque d'autres solutions sont prévues pour éviter ces interférences entre les ensembles émetteur-récepteur (10 ; 20).

Ainsi, le système de contrôle est de préférence configuré pour que les sources lumineuses (11 ; 21) émettent en même temps, c'est-à-dire de façon concomitante. Cela permet d'avoir par exemple des mesures en continue, ce qui permet de se rapprocher au mieux d'une détection en temps réel et en continue. Cela permet aussi d'augmenter la fiabilité de la détection puisqu'il est possible de corréler la détection des deux capteurs lumineux (12 ; 22) au même temps t, et pas l'une à un temps t et l'autre à un temps t+n. La corrélation en est en outre simplifiée. Le système de contrôle de l'appareil 1 peut ainsi comprendre des moyens de synchronisation des sources lumineuses (11 ; 21), le système de contrôle étant donc configuré pour synchroniser l'émission des sources lumineuses (11 ; 21).

Comme on va le voir en détail plus loin, il peut être avantageux de modifier la puissance d'émission des sources lumineuses (11 ; 21) au cours du processus de détermination du taux d'hématocrite du liquide en circulation dans la portion tubulaire 2. A cette fin, le système de contrôle peut donc comprendre des moyens de modification de la puissance émise par les sources lumineuses (11 ; 21), le système de contrôle étant donc configuré pour modifier la puissance émise par les sources lumineuses (11 ; 21). Cette modification de la puissance d'émission des sources lumineuses (11 ; 21) peut par exemple dépendre de la valeur du taux d'hématocrite détecté pour le liquide en circulation dans la portion tubulaire 2.

### *Fonctionnement de l'appareil pour la détermination du taux d'hématocrite d'un liquide en circulation*

Les signaux lumineux reçus par les capteurs lumineux (12 ; 22) des ensembles émetteur-récepteur (10 ; 20) sont destinés à être traités par le système de traitement pour déterminer le taux d'hématocrite du liquide en circulation dans la portion tubulaire autour de laquelle l'appareil 1 a été positionné.

L'appareil 1 pour la détermination du taux d'hématocrite d'un liquide en circulation fonctionne donc selon les étapes suivantes :

- émission de faisceaux lumineux en direction de la portion tubulaire avec les sources lumineuses qui sont configurées pour émettre des faisceaux lumineux selon une longueur d'onde d'émission choisie pour correspondre à un point isobestique de l'hémoglobine ;
- réception des signaux lumineux transmis à travers la portion tubulaire et le liquide en circulation avec les capteurs lumineux étant associés aux sources lumineuses respectivement ;
- détermination du taux d'hématocrite mesuré pour le liquide par un traitement des signaux lumineux reçus par les capteurs, notamment par un calcul effectué par le système de traitement.

Il existe différentes méthodes de calcul corrélatif pour déterminer le taux d'hématocrite en fonction des signaux lumineux issus de sources lumineuses (11 ; 21) émettant selon longueur d'onde d'émission choisie pour correspondre à un point isobestique de l'hémoglobine.

Il est par exemple possible d'utiliser la formule proposée dans l'article intitulé « Noninvasive and Continuous Hematocrit Measurement by Optical Method without Calibration » publié par SHOTA EKUNI et YOSHIYUKI SANKAI dans « Electronics and Communications in Japan, Vol. 99, No. 9, 2016 ».

Selon cette méthode, le taux d'hématocrite est calculé comme suit : il est connu que la concentration d'une substance absorbant la lumière et l'intensité de la lumière transmise en traversant la substance ont une relation logarithmique. La présente méthode s'applique pour une mesure en diffusion en intégrant les deux ensembles émetteur-récepteur (10 ; 20) fonctionnant aux longueurs d'ondes $\lambda 1$ et $\lambda 2$ et permet de déterminer la valeur de la variable $D_{pw}$ selon la formule suivante :

$$D_{pw} = \log_{10}\left(\frac{I}{I - \Delta I}\right)_{\lambda 1} - \log_{10}\left(\frac{I}{I - \Delta I}\right)_{\lambda 2}$$

- Où $\Delta I$ est la différence de la transmission de l'intensité lumineuse entre les deux récepteurs ;
- Où $[\log_{10}(I / (I - \Delta I)_{\lambda 1}]$ et $[\log_{10}(I / (I - \Delta I)_{\lambda 2}]$ sont les différences de l'intensité de la lumière diffusée aux longueurs d'onde $\lambda 1$ et $\lambda 2$.

La valeur obtenue $D_{pw}$ est fonction du niveau d'hématocrite de façon linéaire.

L'adaptation de cette formule à l'appareil de détermination du taux d'hématocrite fonctionnant en transmission permet de déterminer la valeur $D_{pw}$ selon par exemple la formule suivante :

$$D_{pw} = \log_{10}\left(\frac{I}{I_0}\right)_{\lambda 1} - \log_{10}\left(\frac{I}{I_0}\right)_{\lambda 2}$$

- Où $I_0$ est la valeur de blanc relevé à la calibration pour les ensembles émetteur-récepteur (10 ; 20) des longueurs d'ondes $\lambda 1$ et $\lambda 2$ ;
- Où $[\text{Log}_{10} I]$ est le logarithme de l'intensité de la lumière transmise pour les longueurs d'ondes $\lambda 1$ et $\lambda 2$.

La valeur obtenue $D_{pw}$ est également fonction du niveau d'hématocrite de façon linéaire.

Il a été constaté que la détermination du taux d'hématocrite par ces méthodes de calcul pouvait varier et par moment manquer de fiabilité en fonction du taux d'hématocrite du liquide en circulation. En particulier, il a été détecté des cas où les calculs pouvaient être faussés pour les faibles taux d'hématocrite (typiquement inférieur à 20%) et/ou pour les hauts taux d'hématocrite (typiquement supérieurs à 50%).

Or il peut être nécessaire d'avoir un appareil 1 de détermination du taux d'hématocrite fonctionnant de manière fiable pour une large gamme de taux d'hématocrite, ce qui est particulièrement avantageux lorsque l'appareil 1 est utilisé par exemple dans un ensemble de traitement de liquide hémorragique où le liquide hémorragique à traiter a généralement un faible taux d'hématocrite (typiquement inférieur à 20%, voire inférieur à 10%) avant de débuter le traitement alors que le taux d'hématocrite cible à atteindre pour le liquide hémorragique traité est élevé, par exemple d'au moins 35%, voire au moins 45%, et parfois au moins 50%.

Pour fiabiliser la détermination du taux d'hématocrite quelle que soit la valeur de ce taux d'hématocrite, il est proposé de pouvoir modifier la puissance d'émission d'au moins une des sources lumineuses (11 ; 21) des ensembles émetteur-récepteur (10 ; 20) au cours de la mesure du taux d'hématocrite en fonction du taux d'hématocrite calculé pour le liquide.

De préférence, le système de contrôle est prévu pour modifier la puissance d'émission de toutes les sources lumineuses (11 ; 21) des ensembles émetteur-récepteur (10 ; 20) au cours de la mesure du taux d'hématocrite en fonction du taux d'hématocrite calculé pour le liquide.

Avantageusement, la puissance d'émission des sources lumineuses (11 ; 21) est contrôlée de manière indépendante pour chacune des sources lumineuses (11 ; 21). Ce contrôle indépendant est particulièrement avantageux lorsque les sources lumineuses (11 ; 21) sont différentes, notamment lorsque les longueurs d'onde d'émission sont différentes.

Il est d'abord à noter qu'il est avantageux de ne pas utiliser les sources lumineuses (11 ; 21) à 100% de leur capacité. En effet, il est préférable d'utiliser les sources lumineuses (11 ; 21) à une puissance d'émission inférieure à la puissance maximum des sources lumineuses (11 ; 21) pour augmenter la longévité de l'appareil 1 d'une part, mais également pour éviter une possible dégradation des éléments de l'appareil 1, ou une chauffe du liquide en circulation à analyser par exemple.

Pour augmenter la sensibilité de l'appareil 1 quelle que soit la valeur du taux d'hématocrite du liquide en circulation, et sans avoir à modifier les paramètres du capteur lumineux (12 ; 22), il est en outre avantageux de faire varier la puissance d'émission des sources lumineuses (11 ; 21) en fonction du taux d'hématocrite. En particulier, plus le taux d'hématocrite est élevé et plus le signal lumineux en provenance des sources lumineuses (11 ; 21) risque d'être absorbé par le liquide en circulation, ce qui peut être compensé par une augmentation de l'intensité d'émission des sources lumineuses (11 ; 21) pour des niveaux d'intensités de réception au niveau des capteurs lumineux (12 ; 22) semblables.

La puissance d'émission des sources lumineuses (11 ; 21) est donc pilotée en fonction du niveau de détection des capteurs lumineux (12 ; 22) corrélé au taux d'hématocrite mesuré.

En particulier, la puissance d'émission des sources lumineuses (11 ; 21) peut être pilotée en fonction du seuil de non-linéarité au-dessous duquel la valeur mesurée par les capteurs lumineux (12 ; 22) ne permet pas de calculer le taux d'hématocrite avec suffisamment de fiabilité.

En particulier, il est avantageux de piloter la puissance d'émission des sources lumineuses (11 ; 21) pour que le signal reçu ait une puissance supérieure au seuil de non-linéarité mais au plus proche de ce seuil de non-linéarité, tout en étant d'un niveau suffisant selon le taux d'hématocrite mesuré.

De manière alternative ou complémentaire, la puissance d'émission des sources lumineuses (11 ; 21) peut être pilotée en fonction du seuil de saturation du capteur lumineux (12 ; 22) au-delà duquel le signal lumineux reçu au niveau du capteur lumineux (12 ; 22) n'est pas mesurable.

En pratique, même s'il est possible d'utiliser chaque source lumineuse (11 ; 21) à 100% de leur niveau de puissance maximum d'émission, il est avantageux d'utiliser une puissance d'émission pour les sources lumineuses (11 ; 21) comprise entre 10% et 60% de la puissance maximum d'émission desdites sources lumineuses.

L'appareil 1 proposé est prévu pour permettre une détermination d'une large gamme de taux d'hématocrite, en particulier à la fois pour des taux d'hématocrite aussi bas que 5%, voire moins que 5%, que pour des taux d'hématocrite hauts, de l'ordre de 50%, voire de l'ordre de 60% ou supérieur.

En cours d'utilisation de l'appareil 1 pour déterminer le taux d'hématocrite d'un liquide en circulation, il est avantageux de faire augmenter progressivement la puissance d'émission d'au moins une des sources lumineuses (11 ; 21), et de préférence de toutes les sources lumineuses (11 ; 21), en particulier au fur et à mesure que le taux d'hématocrite du liquide augmente. De préférence, la puissance d'émission d'au moins une des sources lumineuses (11 ; 21) est augmentée à partir d'une valeur seuil du taux d'hématocrite mesuré pour le liquide.

Lorsque le taux d'hématocrite mesuré est inférieur à 30%, la puissance d'émission d'au moins une des sources lumineuses (11 ; 21), et de préférence de toutes les sources lumineuses (11 ; 21), peut par exemple être fixée à une valeur comprise entre 10% et 30%, de préférence sensiblement égale à 20%, de la puissance maximum d'émission de la source lumineuse correspondante.

Lorsque le taux d'hématocrite mesuré est supérieur ou égal à 30%, la puissance d'émission d'au moins une des sources lumineuses (11 ; 21), et de préférence de toutes les sources lumineuses (11 ; 21), peut par exemple être fixée à une valeur comprise entre 30% et 100%, de préférence sensiblement égale à 50%, de la puissance maximum d'émission de la source lumineuse correspondante.

Selon un mode particulier de réalisation, la puissance d'émission d'au moins une des sources lumineuses (11 ; 21), et de préférence de toutes les sources lumineuses (11 ; 21), est réglée de sorte que :

- la puissance d'émission de ladite source lumineuse est à un premier niveau de puissance pour des valeurs du taux d'hématocrite mesuré pour le liquide inférieures à une première valeur seuil ;
- la puissance d'émission de ladite source lumineuse est à un deuxième niveau de puissance pour des valeurs du taux d'hématocrite mesuré pour le liquide supérieures ou égales à la première valeur seuil mais inférieure à une deuxième valeur seuil supérieure à la première valeur seuil ; et
- la puissance d'émission de ladite source lumineuse est à un troisième niveau de puissance pour des valeurs du taux d'hématocrite mesuré pour le liquide supérieures ou égales à la deuxième valeur seuil.

Selon ce mode de réalisation, un exemple spécifique de contrôle de la ou des sources lumineuses (11 ; 21) est le suivant :

- la puissance d'émission de la source lumineuse est fixée à une valeur comprise entre 5% et 15%, de préférence égale à 10%, de la puissance maximum d'émission de ladite source lumineuse lorsque le taux d'hématocrite mesuré est inférieur à 20% ;
- la puissance d'émission de la source lumineuse est fixée à une valeur comprise entre 15% et 30%, de préférence égale à 20%, de la puissance maximum d'émission de ladite source lumineuse lorsque le taux d'hématocrite mesuré est compris entre 20% et 30% ; et
- la puissance d'émission de la source lumineuse est fixée à une valeur comprise entre 30% et 100%, de préférence égale à 55%, de la puissance maximum d'émission de ladite source lumineuse lorsque le taux d'hématocrite mesuré est supérieur ou égal à 30%.

La puissance d'émission des sources lumineuses peut également être modifiée au cours de la mesure du taux d'hématocrite en fonction de la présence ou non de liquide dans la portion tubulaire 2 et/ou de la nature dudit liquide. En particulier, si la portion tubulaire est dépourvue de liquide, il est préférable que la ou les sources lumineuses (11 ; 21) soient maintenu à un niveau d'émission minimum, voire nul.

REFERENCES BIBLIOGRAPHIQUES

- « Noninvasive and Continuous Hematocrit Measurement by Optical Method without Calibration » de SHOTA EKUNI et YOSHIYUKI SANKAI (Electronics and Communications in Japan, Vol. 99, No. 9, 2016)

**Revendications**

1. Procédé de détermination du taux d'hématocrite et/ou du taux d'hémoglobine d'un liquide en circulation dans une portion tubulaire (2), comprenant :

   - l'émission de faisceaux lumineux en direction de la portion tubulaire (2) avec au moins deux sources lumineuses (11 ; 21), chacune des deux sources lumineuses (11 ; 21) étant configurée pour émettre des faisceaux lumineux selon une longueur d'onde d'émission choisie pour correspondre à un point isobestique de l'hémoglobine ;
   - la réception de signaux lumineux transmis à travers la portion tubulaire (2) avec au moins deux capteurs lumineux (12 ; 22), chaque capteur lumineux (12 ; 22) étant associé à l'une des deux sources lumineuses (11 ; 21) ;
   - calcul du taux d'hématocrite et/ou du taux d'hémoglobine du liquide par un traitement des signaux lumineux reçus par les capteurs lumineux (12 ; 22) ;

   **caractérisé en ce que** la puissance d'émission d'au moins une des sources lumineuses (11 ; 21) est modifiée au cours de la détermination du taux d'hématocrite et/ou du taux d'hémoglobine en fonction du taux d'hématocrite et/ou respectivement du taux d'hémoglobine calculé pour le liquide.

2. Procédé selon la revendication 1, dans lequel la puissance d'émission utilisée pour les sources lumineuses (11 ; 21) est au maximum égale à 100% de la puissance maximum d'émission desdites sources lumineuses (11 ; 21), et de préférence comprise entre 10% et 60% de la puissance maximum d'émission desdites sources lumineuses (11 ; 21).

3. Procédé selon l'une quelconque des revendications 1 et 2, dans lequel la puissance d'émission des sources lumineuses (11 ; 21) est contrôlée de manière indépendante pour chacune des sources lumineuses (11 ; 21).

4. Procédé selon l'une quelconque des revendications 1 à 3, dans lequel la puissance d'émission d'au moins une des sources lumineuses (11 ; 21) est augmentée à partir d'une valeur seuil du taux d'hématocrite et/ou du taux d'hémoglobine calculé pour le liquide ; dans lequel de préférence :

- la puissance d'émission de la source lumineuse (11 ; 21) est fixée à une valeur comprise entre 10% et 30%, de préférence égale à 20%, de la puissance maximum d'émission de ladite source lumineuse (11 ; 21) lorsque le taux d'hématocrite calculé est inférieur à 30% ; et

- la puissance d'émission de la source lumineuse (11 ; 21) est fixée à une valeur comprise entre 30% et 100%, de préférence égale à 55%, de la puissance maximum d'émission de ladite source lumineuse (11 ; 21) lorsque le taux d'hématocrite calculé est supérieur ou égal à 30%.

5. Procédé selon l'une quelconque des revendications 1 à 4, dans lequel la puissance d'émission des sources lumineuses (11 ; 21) est modifiée au cours de la détermination du taux d'hématocrite et/ou du taux d'hémoglobine en fonction de la présence ou non de liquide dans la portion tubulaire (2) et/ou de la nature dudit liquide.

6. Procédé selon l'une quelconque des revendications 1 à 5, dans lequel les sources lumineuses (11 ; 21) sont contrôlées pour émettre des faisceaux lumineux de façon concomitante.

7. Appareil de détermination du taux d'hématocrite et/ou du taux d'hémoglobine d'un liquide en circulation dans une portion tubulaire (2), comprenant :

deux ensembles émetteur-récepteur (10 ; 20), chaque ensemble émetteur-récepteur (10 ; 20) comprenant une source lumineuse (11 ; 21) et un capteur lumineux (12 ; 22) prévus pour être agencés de part et d'autre de la portion tubulaire (2) au niveau d'une zone de circulation du liquide pour une mesure en transmission ;
la source lumineuse (11 ; 21) de chacun des deux ensembles émetteur-récepteur (10 ; 20) étant configurée pour émettre des faisceaux lumineux selon une longueur d'onde d'émission choisie pour correspondre à un point isobestique de l'hémoglobine ;
un système de traitement programmé pour déterminer le taux d'hématocrite et/ou du taux d'hémoglobine du liquide en fonction des signaux lumineux reçus par les capteurs lumineux (12 ; 22) des ensembles émetteur-récepteur (10 ; 20) ; et
un système de contrôle comprenant des moyens de modification de la puissance émise par les sources lumineuses (11 ; 21), le système de contrôle étant programmé pour modifier la puissance d'émission des sources lumineuses (11 ; 21) en fonction du taux d'hématocrite et/ou du taux d'hémoglobine déterminé pour le liquide.

8. Appareil selon la revendication 7, comprenant un ensemble support (30) sur lequel sont montés les deux ensembles émetteur-récepteur (10 ; 20), l'ensemble support (30) étant prévu pour être positionné autour de la portion tubulaire (2) ;

dans lequel les ensembles émetteur-récepteur (10 ; 20) sont de préférence assemblés sur un unique support (31) ayant une rainure (32) destinée à recevoir la portion tubulaire (2) ;
l'appareil comprenant de préférence en outre un couvercle (33) prévu pour recouvrir au moins partiellement la rainure (32), ledit couvercle (33) comprenant une portion de compression destinée à maintenir en position la portion tubulaire (2) positionnée dans la rainure (32).

9. Appareil selon l'une quelconque des revendications 7 et 8, dans lequel les sources lumineuses (11 ; 21) respectives des deux ensembles émetteur-récepteur (10 ; 20) sont configurées pour émettre des faisceaux lumineux à deux longueurs d'onde d'émission différentes.

10. Appareil selon l'une quelconque des revendications 7 à 9, dans lequel au moins une des sources lumineuses (11 ; 21) des ensembles émetteur-récepteur (10 ; 20) est configurée pour émettre des faisceaux lumineux selon une longueur d'onde d'émission choisie pour une absorption des faisceaux lumineux sensiblement identique dans de l'eau ou dans du plasma.

11. Appareil selon l'une quelconque des revendications 7 à 10, dans lequel au moins un, et de préférence chaque, système de collimation (13 ; 23) comprend :

- un ensemble de lentille(s) amont (131 ; 231) ayant un plan focal et étant positionné entre la source lumineuse (11 ; 21) et le capteur lumineux (12 ; 22) correspondants du côté de la source lumineuse (11 ; 21) par rapport à la portion tubulaire (2), la source lumineuse (11 ; 21) étant positionnée à plus ou moins 10 mm du plan focal de l'ensemble de lentille(s) amont (131 ; 231), et de préférence dans plan focal de l'ensemble de lentille(s) amont (131 ; 231) ; et/ou
- un ensemble de lentille(s) aval (132 ; 232) ayant un plan focal et étant positionné entre la source lumineuse (11 ; 21) et le capteur lumineux (12 ; 22) correspondants du côté du capteur lumineux (12 ; 22) par rapport à la portion tubulaire (2), le capteur lumineux (12 ; 22) étant positionné à plus ou moins 10 mm du plan focal de l'ensemble de lentille(s) aval (132 ; 232), et de préférence dans plan focal de l'ensemble de lentille(s) aval (132 ; 232) ; et /ou

- un ensemble de lentille(s) aval (132 ; 232) ayant un plan focal et étant positionné entre la source lumineuse (11 ; 21) et le capteur lumineux (12 ; 22) correspondants du côté du capteur lumineux (12 ; 22) par rapport à la portion tubulaire (2), l'ensemble de lentille(s) aval (132 ; 232) est positionné de sorte que les faisceaux lumineux en sortie de la paroi de sortie (202) de la portion tubulaire (2) convergent à plus ou moins 10 mm du plan focal de l'ensemble de lentille(s) aval, et de préférence dans plan focal de l'ensemble de lentille(s) aval (132 ; 232) ; et/ou

- un diaphragme amont (133 ; 233) positionné entre la source lumineuse (11 ; 21) et le capteur lumineux (12 ; 22) correspondants du côté de la source lumineuse (11 ; 21) par rapport à la portion tubulaire (2), le diaphragme amont (133 ; 233) étant prévu pour laisser passer une portion centrale des faisceaux lumineux émis par la source lumineuse (11 ; 21) en direction du capteur lumineux (12 ; 22) et pour stopper une portion périphérique des faisceaux lumineux émis par la source lumineuse (11 ; 21) ; et/ou

- un diaphragme aval (134 ; 234) positionné entre la source lumineuse (11 ; 21) et le capteur lumineux (12 ; 22) correspondants du côté du capteur lumineux (12 ; 22) par rapport à la portion tubulaire (2), le diaphragme aval (134 ; 234) étant prévu pour laisser passer une portion centrale des faisceaux lumineux transmis à travers la portion tubulaire (2) en direction du capteur lumineux (12 ; 22) et pour stopper une portion périphérique des faisceaux lumineux transmis à travers la portion tubulaire (2) ; et/ou

- un filtre amont (135 ; 235) positionné entre la source lumineuse (11 ; 21) et le capteur lumineux (12 ; 22) correspondants du côté de la source lumineuse (11 ; 21) par rapport à la portion tubulaire (2), et/ou un filtre aval (136 ; 236) positionné entre la source lumineuse (11 ; 21) et le capteur lumineux (12 ; 22) correspondants du côté du capteur lumineux (12 ; 22) par rapport à la portion tubulaire (2), les filtres amont (135 ; 235) et aval (136 ; 236) du système de collimation (13 ; 23) d'un ensemble émetteur-récepteur (10 ; 20) étant prévus pour filtrer au moins la longueur d'onde d'émission de la source lumineuse (11 ; 21) de l'autre ensemble émetteur-récepteur (10 ; 20).

12. Appareil selon l'une quelconque des revendications 7 à 11, dans lequel

   - la source lumineuse (11 ; 21) d'un premier des deux ensembles émetteur-récepteur (10 ; 20) est configurée pour émettre des faisceaux lumineux à une longueur d'onde comprise entre 780 nm et 840 nm, de préférence comprise entre 800 nm et 820 nm, et de préférence encore égale à 810 nm ; et

   - la source lumineuse (11 ; 21) d'un deuxième des deux ensembles émetteur-récepteur (10 ; 20) est configurée pour émettre des faisceaux lumineux à une longueur d'onde comprise entre 1270 nm et 1330 nm, de préférence comprise entre 1290 nm et 1310 nm, et de préférence encore égale à 1300 nm.

13. Appareil selon l'une quelconque des revendications 7 à 12, dans lequel les sources lumineuses (11 ; 21) des ensembles émetteur-récepteur (10 ; 20) sont positionnés du même côté par rapport à la portion tubulaire (2).

14. Appareil selon l'une quelconque des revendications 7 à 13, dans lequel le système de contrôle comprend en outre des moyens de synchronisation des sources lumineuses (11 ; 21).

15. Appareil selon l'une quelconque des revendications 7 à 14, prévu pour une détermination du taux d'hématocrite et/ou du taux d'hémoglobine sans déformation de la portion tubulaire (2).

16. Appareil selon l'une quelconque des revendications 7 à 14, comprenant un système de déformation de la portion tubulaire (2) en regard des ensembles émetteur-récepteur (10 ; 20), le système de déformation étant prévu pour déformer une section circulaire de la portion tubulaire (2) en une section ellipsoïdale ;

   dans lequel de préférence les sources lumineuses (11 ; 21) et tous éléments des ensembles émetteur-récepteur (10 ; 20) prévus pour être du côté de la source lumineuse (11 ; 21) correspondante par rapport à la portion tubulaire (2) sont positionnés d'un côté d'un grand axe définissant la section ellipsoïdale, et les capteurs lumineux (12 ; 22) et tous éléments des ensembles émetteur-récepteur (10 ; 20) prévus pour être du côté du capteur lumineux (12 ; 22) correspondant par rapport à la portion tubulaire (2) sont positionnés de l'autre côté du grand axe définissant la section ellipsoïdale ;

   dans lequel de préférence encore la section ellipsoïdale est définie par un grand rayon (Ra) le long du grand axe et par un petit rayon (Rb) le long d'un petit axe perpendiculaire au grand axe, la section ellipsoïdale ayant, dans un état déformé de la portion tubulaire (2), un petit rayon (Rb) ayant une longueur comprise entre 30% et 70%, et de préférence de l'ordre de 50%, du rayon de la section circulaire de la portion tubulaire (2) en état non-déformé.

**Patentansprüche**

1. Verfahren zur Bestimmung des Hämatokritwertes und/oder des Hämoglobinwertes einer in einem Schlauchabschnitt (2) fließenden Flüssigkeit, umfassend:

   - das Senden von Lichtstrahlen in Richtung des Schlauchabschnitts (2) mit mindestens zwei Lichtquellen (11; 21), wobei jede der zwei Lichtquellen (11; 21) dazu ausgelegt ist, Lichtstrahlen in einer Sendewellenlänge zu senden, die so gewählt ist, dass sie einem isobestischen Punkt des Hämoglobins entspricht;
   - das Empfangen von durch den Schlauchabschnitt (2) übertragenen Lichtsignalen mit mindestens zwei Lichtsensoren (12; 22), wobei jeder Lichtsensor (12; 22) einer der zwei Lichtquellen (11; 21) zugeordnet ist;
   - Berechnen des Hämatokritwertes und/oder des Hämoglobinwertes der Flüssigkeit durch eine Verarbeitung der von den Lichtsensoren (12; 22) empfangenen Lichtsignale;

   **dadurch gekennzeichnet, dass** die Sendeleistung von mindestens einer der Lichtquellen (11; 21) während der Bestimmung des Hämatokritwertes und/oder des Hämoglobinwertes in Abhängigkeit vom Hämatokritwert bzw. vom Hämoglobinwert verändert wird, der für die Flüssigkeit berechnet wurde.

2. Verfahren nach Anspruch 1, wobei die für die Lichtquellen (11; 21) verwendete Sendeleistung maximal gleich 100% der maximalen Sendeleistung der Lichtquellen (11; 21) ist, und vorzugsweise zwischen 10% und 60% der maximalen Sendeleistung der Lichtquellen (11; 21) liegt.

3. Verfahren nach einem der Ansprüche 1 und 2, wobei die Sendeleistung der Lichtquellen (11; 21) für jede der Lichtquellen (11; 21) unabhängig gesteuert wird.

4. Verfahren nach einem der Ansprüche 1 bis 3, wobei die Sendeleistung von mindestens einer der Lichtquellen (11; 21) ausgehend von einem Schwellenwert des Hämatokritwertes und/oder des Hämoglobinwertes erhöht wird, der für die Flüssigkeit berechnet wurde;
   und wobei vorzugsweise:

   - die Sendeleistung der Lichtquelle (11; 21) auf einen Wert festgelegt ist, der zwischen 10% und 30%, vorzugsweise bei gleich 20%, der maximalen Sendeleistung der Lichtquelle (11; 21) liegt, wenn der berechnete Hämatokritwert unter 30% beträgt; und
   - die Sendeleistung der Lichtquelle (11; 21) auf einen Wert festgelegt ist, der zwischen 30% und 100%, vorzugsweise bei gleich 55%, der maximalen Sendeleistung der Lichtquelle (11; 21) liegt, wenn der berechnete Hämatokritwert über oder gleich 30% beträgt.

5. Verfahren nach einem der Ansprüche 1 bis 4, wobei die Sendeleistung der Lichtquellen (11; 21) während der Bestimmung des Hämatokritwertes und/oder des Hämoglobinwertes in Abhängigkeit von der Anwesenheit von Flüssigkeit oder nicht im Schlauchabschnitt (2) und/oder von der Art der Flüssigkeit verändert wird.

6. Verfahren nach einem der Ansprüche 1 bis 5, wobei die Lichtquellen (11; 21) gesteuert werden, um Lichtstrahlen gleichzeitig zu senden.

7. Gerät zur Bestimmung des Hämatokritwertes und/oder des Hämoglobinwertes einer in einem Schlauchabschnitt (2) fließenden Flüssigkeit, umfassend:

   zwei Sender-Empfänger-Anordnungen (10; 20), wobei jede Sender-Empfänger-Anordnung (10; 20) eine Lichtquelle (11; 21) und einen Lichtsensor (12; 22) umfasst, die zur beidseitigen Anordnung am Schlauchabschnitt (2) im Bereich einer Fließzone der Flüssigkeit für eine Transmissionsmessung vorgesehen sind;
   wobei die Lichtquelle (11; 21) jeder der zwei Sender-Empfänger-Anordnungen (10; 20) zum Senden der Lichtstrahlen in einer Sendewellenlänge ausgelegt ist, die so gewählt ist, dass sie einem isobestischen Punkt des Hämoglobins entspricht;
   ein Verarbeitungssystem, das programmiert ist, um den Hämatokritwert und/oder den Hämoglobinwert der Flüssigkeit in Abhängigkeit von den von den Lichtsensoren (12; 22) der Sender-Empfänger-Anordnungen (10; 20) empfangenen Lichtsignalen zu bestimmen; und
   ein Steuerungssystem, das Mittel zum Ändern der von den Lichtquellen (11; 21) gesendeten Leistungen umfasst, wobei das Steuerungssystem programmiert ist, um die Sendeleistung der Lichtquellen (11; 21) in Abhängigkeit vom Hämatokritwert und/oder vom Hämoglobinwert zu ändern, der für die Flüssigkeit bestimmt wurde.

8. Gerät nach Anspruch 7, umfassend eine Trägeranordnung (30), auf der die zwei Sender-Empfänger-Anordnungen (10; 20) angebracht sind, wobei die Trägeranordnung (30) zur Positionierung um den Schlauchabschnitt (2) vorgesehen ist;

   wobei die Sender-Empfänger-Anordnungen (10; 20) vorzugsweise auf einem einzigen Träger (31) zusammengebaut sind, der eine Rille (32) hat, die zum Empfang des Schlauchabschnitts (2) bestimmt ist;
wobei das Gerät vorzugsweise ferner einen Deckel (33) umfasst, der vorgesehen ist, die Rille (32) wenigstens teilweise abzudecken, wobei der Deckel (33) einen Kompressionsabschnitt umfasst, der bestimmt ist, den in der Rille (32) positionierten Schlauchabschnitt (2) in Position zu halten.

9. Gerät nach einem der Ansprüche 7 und 8, wobei die jeweiligen Lichtquellen (11; 21) der zwei Sender-Empfänger-Anordnungen (10; 20) dazu ausgelegt sind, Lichtstrahlen in zwei unterschiedlichen Sendewellenlängen zu senden.

10. Gerät nach einem der Ansprüche 7 bis 9, wobei mindestens eine der Lichtquellen (11; 21) der Sender-Empfänger-Anordnungen (10; 20) dazu ausgelegt ist, Lichtstrahlen in einer Sendewellenlänge zu senden, die für eine Absorption der Lichtstrahlen gewählt ist, die im Wasser oder im Plasma etwa identisch ist.

11. Gerät nach einem der Ansprüche 7 bis 10, wobei mindestens ein und vorzugsweise jedes Kollimationssystem (13; 23) umfasst:

   - eine vorgelagerte Linsenanordnung (131; 231), die eine Fokalebene hat und zwischen der entsprechenden Lichtquelle (11; 21) und dem entsprechenden Lichtsensor (12; 22) auf der Seite der Lichtquelle (11; 21) im Verhältnis zum Schlauchabschnitt (2) positioniert ist, wobei die Lichtquelle (11; 21) in mehr oder weniger als 10 mm von der Fokalebene der vorgelagerten Linsenanordnung (131; 231) und vorzugsweise in der Fokalebene der vorgelagerten Linsenanordnung (131; 231) positioniert ist; und/oder
   - eine nachgelagerte Linsenanordnung (132; 232), die eine Fokalebene hat und zwischen der entsprechenden Lichtquelle (11; 21) und dem entsprechenden Lichtsensor (12; 22) auf der Seite des Lichtsensors (12; 22) im Verhältnis zum Schlauchabschnitt (2) positioniert ist, wobei der Lichtsensor (12; 22) in mehr oder weniger als 10 mm von der Fokalebene der nachgelagerten Linsenanordnung (132; 232) und vorzugsweise in der Fokalebene der nachgelagerten Linsenanordnung (132; 232) positioniert ist; und/oder
   - eine nachgelagerte Linsenanordnung (132; 232), die eine Fokalebene hat und zwischen der entsprechenden Lichtquelle (11; 21) und dem entsprechenden Lichtsensor (12; 22) auf der Seite des Lichtsensors (12; 22) im Verhältnis zum Schlauchabschnitt (2) positioniert ist, wobei die nachgelagerte Linsenanordnung (132; 232) derart positioniert ist, dass die aus der Ausgangswand (202) des Schlauchabschnitts (2) austretenden Lichtstrahlen in mehr oder weniger als 10 mm von der Fokalebene der nachgelagerten Linsenanordnung und vorzugsweise in der Fokalebene der nachgelagerten Linsenanordnung (132; 232) konvergieren; und/oder
   - eine vorgelagerte Blende (133; 233), die zwischen der entsprechenden Lichtquelle (11; 21) und dem entsprechenden Lichtsensor (12; 22) auf der Seite der Lichtquelle (11; 21) im Verhältnis zum Schlauchabschnitt (2) positioniert ist, wobei die vorgelagerte Blende (133; 233) vorgesehen ist, um einen zentralen Abschnitt der von der Lichtquelle (11; 21) in Richtung des Lichtsensors (12; 22) gesendeten Lichtstrahlen hindurchzulassen und um einen peripheren Abschnitt der von der Lichtquelle (11; 21) gesendeten Lichtstrahlen zu stoppen; und/oder
   - eine nachgelagerte Blende (134; 234), die zwischen der entsprechenden Lichtquelle (11; 21) und dem entsprechenden Lichtsensor (12; 22) auf der Seite des Lichtsensors (12; 22) im Verhältnis zum Schlauchabschnitt (2) positioniert ist, wobei die nachgelagerte Blende (134; 234) vorgesehen ist, um einen zentralen Abschnitt der durch den Schlauchabschnitt (2) in Richtung des Lichtsensors (12; 22) gesendeten Lichtstrahlen hindurchzulassen und um einen peripheren Abschnitt der durch den Schlauchabschnitt (2) übertragenen Lichtstrahlen zu stoppen; und/oder
   - einen vorgelagerten Filter (135; 235), der zwischen der entsprechenden Lichtquelle (11; 21) und dem entsprechenden Lichtsensor (12; 22) auf der Seite der Lichtquelle (11; 21) im Verhältnis zum Schlauchabschnitt (2) positioniert ist, und/oder einen nachgelagerten Filter (136; 236), der zwischen der entsprechenden Lichtquelle (11; 21) und dem entsprechende Lichtsensor (12; 22) auf der Seite des Lichtsensors (12; 22) im Verhältnis zum Schlauchabschnitt (2) positioniert ist, wobei der vorgelagerte Filter (135; 235) und der nachgelagerte Filter (136; 236) des Kollimationssystems (13; 23) einer Sender-Empfänger-Anordnung (10; 20) vorgesehen sind, um mindestens die Sendewellenlänge der Lichtquelle (11; 21) der anderen Sender-Empfänger-Anordnung (10; 20) zu filtern.

12. Gerät nach einem der Ansprüche 7 bis 11, wobei

   - die Lichtquelle (11; 21) einer ersten der zwei Sender-Empfänger-Anordnungen (10; 20) dazu ausgelegt ist, Lichtstrahlen in einer Wellenlänge zu senden, die zwischen 780 nm und 840 nm, vorzugsweise zwischen 800 nm und 820 nm liegt und ebenfalls vorzugsweise gleich 810 nm beträgt; und

- die Lichtquelle (11; 21) einer zweiten der zwei Sender-Empfänger-Anordnungen (10; 20) dazu ausgelegt ist, Lichtstrahlen in einer Wellenlänge zu senden, die zwischen 1270 nm und 1330 nm, vorzugsweise zwischen 1290 nm und 1310 nm liegt und ebenfalls vorzugsweise gleich 1300 nm beträgt.

**13.** Gerät nach einem der Ansprüche 7 bis 12, wobei die Lichtquellen (11; 21) der Sender-Empfänger-Anordnungen (10; 20) auf derselben Seite im Verhältnis zum Schlauchabschnitt (2) positioniert sind.

**14.** Gerät nach einem der Ansprüche 7 bis 13, wobei das Steuerungssystem ferner Synchronisationsmittel der Lichtquellen (11; 21) umfasst.

**15.** Gerät nach einem der Ansprüche 7 bis 14, das für eine Bestimmung des Hämatokritwertes und/oder des Hämoglobinwertes ohne Verformung des Schlauchabschnitts (2) vorgesehen ist.

**16.** Gerät nach einem der Ansprüche 7 bis 14, umfassend ein Verformungssystem des den Sender-Empfänger-Anordnungen (10; 20) zugewandten Schlauchabschnitts (2), wobei das Verformungssystem vorgesehen ist, einen kreisförmigen Querschnitt des Schlauchabschnitts (2) in einen ellipsenförmigen Querschnitt zu verformen;

wobei vorzugsweise die Lichtquellen (11; 21) und alle Elemente der Sender-Empfänger-Anordnungen (10; 20), die vorgesehen sind, an der Seite der entsprechenden Lichtquelle (11; 21) im Verhältnis zum Schlauchabschnitt (2) zu sein, auf einer Seite einer großen Achse positioniert sind, die den ellipsenförmigen Querschnitt definiert, und die Lichtsensoren (12; 22) und alle Elemente der Sender-Empfänger-Anordnungen (10; 20), die vorgesehen sind, an der Seite des entsprechenden Lichtsensors (12; 22) im Verhältnis zum Schlauchabschnitt (2) zu sein, auf der anderen Seite der großen Achse positioniert sind, die den ellipsenförmigen Querschnitt definiert, wobei ebenfalls vorzugsweise der ellipsenförmige Querschnitt durch einen großen Radius (Ra) entlang der großen Achse und durch einen kleinen Radius (Rb) entlang einer kleinen Achse senkrecht zur großen Achse definiert ist, wobei der ellipsenförmige Querschnitt in einem verformten Zustand des Schlauchabschnitts (2) einen kleinen Radius (Rb) hat, der eine Länge hat, die zwischen 30% und 70% liegt und vorzugsweise zirka 50% des Radius des kreisförmigen Querschnitts des Schlauchabschnitts (2) in nicht-verformtem Zustand beträgt.

**Claims**

**1.** A method for determining the hematocrit level and/or the hemoglobin level of a fluid circulating in a tubular portion (2), comprising:

- the emission of light beams in the direction of the tubular portion (2) with at least two light sources (11; 21), each of the two light sources (11; 21) being configured to emit light beams according to an emission wavelength chosen to correspond to an isosbestic point of hemoglobin;
- the receipt of light signals transmitted through the tubular portion (2) with at least two light sensors (12; 22), each light sensor (12; 22) being associated with one of the two light sources (11; 21);
- the calculation of the hematocrit level and/or the hemoglobin level of the fluid by a processing of the light signals received by the light sensors (12; 22);

**characterized in that** the emission power of at least one of the light sources (11; 21) is modified during the determination of the hematocrit level and/or the hemoglobin level as a function of the hematocrit level and/or of the hemoglobin level respectively calculated for the fluid.

**2.** The method of claim 1, wherein the emission power used for the light sources (11; 21) is at most equal to 100% of the maximum emission power of said light sources (11; 21), and preferably comprised between 10% and 60% of the maximum emission power of said light sources (11; 21).

**3.** The method of any one of claims 1 and 2, wherein the emission power of the light sources (11; 21) is monitored independently for each of the light sources (11; 21).

**4.** The method of any one of claims 1 to 3, wherein the emission power of at least one of the light sources (11; 21) is increased from a threshold value of the hematocrit level and/or the hemoglobin level calculated for the fluid; wherein preferably:

- the emission power of the light source (11; 21) is set to a value comprised between 10% and 30%, preferably equal to 20%, of the maximum emission power of said light source (11; 21) when the calculated hematocrit level is lower than 30%; and
- the emission power of the light source (11; 21) is set to a value comprised between 30% and 100%, preferably equal to 55%, of the maximum emission power of said light source (11; 21) when the calculated hematocrit level is higher than or equal to 30%.

5. The method of any one of claims 1 to 4, wherein the emission power of the light sources (11; 21) is modified during the determination of the hematocrit level and/or the hemoglobin level depending on the presence or absence of fluid in the tubular portion (2) and/or on the nature of said fluid.

6. The method of any one of claims 1 to 5, wherein the light sources (11; 21) are monitored to emit light beams concomitantly.

7. An apparatus for determining the hematocrit level and/or the hemoglobin level of a fluid circulating in a tubular portion (2), comprising:

   two transceiver assemblies (10; 20), each transceiver assembly (10; 20) comprising a light source (11; 21) and a light sensor (12; 22) provided to be arranged on either side of the tubular portion (2) at a fluid circulation area for a measurement in transmission;
   the light source (11; 21) of each of the two transceiver assemblies (10; 20) being configured to emit light beams according to an emission wavelength chosen to correspond to an isosbestic point of hemoglobin;
   a processing system programmed to determine the hematocrit level and/or the hemoglobin level of the fluid as a function of the light signals received by the light sensors (12; 22) of the transceiver assemblies (10; 20); and
   a monitoring system comprising means for modifying the power emitted by the light sources (11; 21), the monitoring system being programmed to modify the emission power of the light sources (11; 21) as a function of the hematocrit level and/or the hemoglobin level determined for the fluid.

8. The apparatus of claim 7, comprising a support assembly (30) on which the two transceiver assemblies (10; 20) are mounted, the support assembly (30) being configured to be positioned around the tubular portion (2);

   wherein the transceiver assemblies (10; 20) are preferably assembled on a single support (31) having a groove (32) intended to receive the tubular portion (2);
   the apparatus preferably further comprising a cover (33) provided to at least partially cover the groove (32), said cover (33) comprising a compression portion intended to hold in position the tubular portion (2) positioned in the groove (32).

9. The apparatus of any one of claims 7 and 8, wherein the respective light sources (11; 21) of the two transceiver assemblies (10; 20) are configured to emit light beams at two different emission wavelengths.

10. The apparatus of any one of claims 7 to 9, wherein at least one of the light sources (11; 21) of the transceiver assemblies (10; 20) is configured to emit light beams according to an emission wavelength chosen for an absorption of the light beams substantially identical in water or in plasma.

11. The apparatus of any one of claims 7 to 10, wherein at least one, and preferably each, collimation system (13; 23) comprises:

   - an upstream lens(es) assembly (131; 231) having a focal plane and being positioned between the corresponding light source (11; 21) and light sensor (12; 22) on the side of the light source (11; 21) with respect to the tubular portion (2), the light source (11; 21) being positioned at more or less 10 mm from the focal plane of the upstream lens(es) assembly (131; 231), and preferably in the focal plane of the upstream lens(es) assembly (131; 231); and/or
   - a downstream lens(es) assembly (132; 232) having a focal plane and being positioned between the corresponding light source (11; 21) and light sensor (12; 22) on the side of the light sensor (12; 22) with respect to the tubular portion (2), the light sensor (12; 22) being positioned at more or less 10 mm from the focal plane of the set of downstream lens(s) (132; 232), and preferably in the focal plane of the set of downstream lens(s) (132; 232); and/or
   - a downstream lens(es) assembly (132; 232) having a focal plane and being positioned between the corresponding light source (11; 21) and light sensor (12; 22) on the side of the light sensor (12; 22) with respect to the tubular portion (2), the downstream lens(es) assembly) (132; 232) is positioned so that the light beams leaving the outlet wall (202) of the tubular portion (2) converge at more or less 10 mm from the focal plane of the downstream lens(es) assembly, and preferably in the focal plane of the downstream lens(es) assembly (132; 232); and/or
   - an upstream diaphragm (133; 233) positioned between the corresponding light source (11; 21) and light sensor (12; 22) on the side of the light source (11; 21) with respect to the tubular portion (2), the upstream diaphragm (133; 233) being provided to let pass a central portion of the light beams emitted by the light source (11; 21) in the direction of the light sensor (12; 22) and to stop a peripheral portion of the light beams emitted by the light source (11; 21); and/or

- a downstream diaphragm (134; 234) positioned between the corresponding light source (11; 21) and light sensor (12; 22) on the side of the light sensor (12; 22) with respect to the tubular portion (2), the downstream diaphragm (134; 234) being provided to let pass a central portion of the light beams transmitted through the tubular portion (2) in the direction of the light sensor (12; 22) and to stop a peripheral portion of the light beams transmitted through the tubular portion (2); and/or

- an upstream filter (135; 235) positioned between the corresponding light source (11; 21) and light sensor (12; 22) on the side of the light source (11; 21) with respect to the tubular portion (2), and/or a downstream filter (136; 236) positioned between the corresponding light source (11; 21) and light sensor (12; 22) on the side of the light sensor (12; 22) with respect to the tubular portion (2), the upstream (135; 235) and downstream (136; 236) filters of the collimation system (13; 23) of a transceiver assembly (10; 20) being provided to filter at least the emission wavelength of the light source (11; 21) of the other transceiver assembly (10; 20).

12. The apparatus of any one of claims 7 to 11, wherein:

- the light source (11; 21) of a first of the two transceiver assemblies (10; 20) is configured to emit light beams at a wavelength comprised between 780 nm and 840 nm, preferably comprised between 800 nm and 820 nm, and more preferably equal to 810 nm; and

- the light source (11; 21) of a second of the two transceiver assemblies (10; 20) is configured to emit light beams at a wavelength comprised between 1,270 nm and 1,330 nm, preferably between 1,290 nm and 1,310 nm, and more preferably equal to 1,300 nm.

13. The apparatus of any one of claims 7 to 12, wherein the light sources (11; 21) of the transceiver assemblies (10; 20) are positioned on the same side with respect to the tubular portion (2).

14. The apparatus of any one of claims 7 to 13, wherein the monitoring system further comprises means for synchronizing the light sources (11; 21).

15. The apparatus of any one of claims 7 to 14, provided for a determination of the hematocrit level and/or the hemoglobin level without deformation of the tubular portion (2).

16. The apparatus of any one of claims 7 to 15, comprising a system for deforming the tubular portion (2) facing the transceiver assemblies (10; 20), the deformation system being provided to deform a circular section of the tubular portion (2) into an ellipsoidal section;

wherein preferably the light sources (11; 21) and all elements of the transceiver assemblies (10; 20) provided to be on the side of the corresponding light source (11; 21) with respect to the tubular portion (2) are positioned on one side of a major axis defining the ellipsoidal section, and the light sensors (12; 22) and all elements of the transceiver assemblies (10; 20) provided to be on the side of the corresponding light sensor (12; 22) with respect to the tubular portion (2) are positioned on the other side of the major axis defining the ellipsoidal section;

wherein more preferably the ellipsoidal section is defined by a major radius (Ra) along the major axis and by a minor radius (Rb) along a minor axis perpendicular to the major axis, the ellipsoidal section having, in a deformed state of the tubular portion (2), a small radius (Rb) having a length comprised between 30% and 70%, and preferably of the order of 50%, of the radius of the circular section of the tubular portion (2) in an undeformed state.

FIG. 1

EP 4 179 318 B1

FIG. 2

FIG. 3

FIG. 4

EP 4 179 318 B1

## FIG. 5

## FIG. 6

# FIG. 7

3114

131

3113   3112   3111

311

3214

231

3213   3212   3211

321

# FIG. 8

312   131   311

31

322   231   321

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **SHOTA EKUNI ; YOSHIYUKI SANKAI.** Noninvasive and Continuous Hematocrit Measurement by Optical Method without Calibration. *Electronics and Communications in Japan,* 2016, vol. 99 (9